# EUROPEAN PATENT SPECIFICATION

(11) **EP 4 406 516 B1**
(45) Date of publication and mention of the grant of the patent: **28.10.2026**
(21) Application number: 24180428.5
(22) Date of filing: 27.01.2020
(51) Int. Cl.: A61F 2/40

(54) **GLENOID IMPLANT**
GLENOIDIMPLANTAT
IMPLANT GLÉNOÏDIEN

(30) Priority: 28.01.2019 US 201962797866 P
(43) Date of publication of application: 31.07.2024
(60) Divisional application: 26206640.0
(62) Document number(s) of the earlier application(s) in accordance with Art. 76 EPC: 20707961.7 / 3 917 457
(73) Proprietor: ENCORE MEDICAL, L.P. (D/B/A DJO SURGICAL), Austin, TX 78758 (US)
(72) Inventor: FRANKLE, Mark, A., Tampa, Florida, 33602 (US); GUTIERREZ, Sergio, Tampa, Florida, 33602 (US); WILLIAMS, Gerald, Villanova, Pennsylvania, 19085 (US)
(74) Representative: Epping - Hermann - Fischer

(56) References cited:
- US-A1- 2007 100 458
- US-A1- 2013 123 929
- US-A1- 2013 150 972
- US-A1- 2016 324 649

## Description

### FIELD OF THE PRESENT DISCLOSURE

The present disclosure relates to glenoid implants for cases of severe bone loss.

### BACKGROUND

Shoulder replacement surgery is currently used to treat patients suffering from disabling pain due to worn or damaged shoulder joints, which can be caused by, e.g., arthritis and/or injury. The humeral implants currently in use are typically made from metal, and the implants are affixed to the bone using bone cement (e.g., polymethylmethacrylate) or by press fitting the implant into the bone using a roughened outer surface coating on the metal for bony integration. Most glenoid (shoulder socket) implants are made completely from polyethylene and affixed to the cortical bone using bone cement. Some glenoid implants have a metal baseplate with a polyethylene insert. Current glenoid implants are made to sit on the surface of a reamed glenoid, which is prepared by removing any remaining cartilage and flattening the bony surface. These implants use either a keel or multiple elongated pegs on the back of the prosthetic glenoid implant to secure the glenoid implant inside the glenoid vault.

Keeled and pegged glenoid implants suffer from several disadvantages, which limit their lifespan once implanted and reduce the number of indications for which they can be used when the age of the patient is a factor. For example, the glenoid implants can loosen due to poor fixation within the bone, and they are prone to wear and fatigue failure of the polyethylene due to adhesion, abrasion, and shear stress. Because of these deficiencies, surgeons hesitate to perform glenoid replacement surgery on young or middle aged patients with glenoid articular cartilage injuries or damage due to early arthritis for fear that the implant may not last more than 10-15 years in the body, thus subjecting the patient to the possibility of two or more surgeries during the lifetime of the patient to preserve the function and pain-free state of the joint. Finally, current glenoid implants with a long keel or pegs are sometimes contraindicated in patients with significant glenoid bone loss. As arthritis progresses, the humeral head can wear medially and destroy the foundation of glenoid bone. In these cases, the glenoid vault can be significantly reduced in volume and depth. Thus, a typical keel or peg design can broach the glenoid vault and injure the suprascapular nerve along the suprascapular notch or spinoglenoid notch with resultant denervation injury to the rotator cuff muscles. Broaching through the glenoid vault can also fracture the body of the scapula and cause early implant loosening.

Because there are more than 20,000 shoulder arthoplasty surgeries performed per year, many U.S. patients incur a risk of continued pain and disability, neuromuscular injuries, or failed shoulder prostheses requiring revision surgery. Thus, there remains a need for an improved glenoid implants. The present disclosure is directed to solving these problems and addressing other needs.
US 2013/150972 A1 discloses a joint prosthesis system, specifically a shoulder prosthesis, for shoulder replacement, revision and repair. The implants provide fixation into the best bone available to a surgeon. The implants are used in a superior-inferior and anterior-posterior construct forming a type of cross or X-shape. The implants allow for interchangeability of the articulating component as well as rotational orientation. The systems allow for augments to accommodate bone loss. The implants allow for additional security using screws or anchors inserted into the scapula.
US 2007/100458 A1 discloses an attachment element for a prosthesis of the shoulder for the articulation of a humerus in a scapula of a shoulder having a glenoid cavity. The prosthesis comprises a first articulation element associated with the top of the humerus, and a second articulation element associated with the glenoid cavity.
US 2013/123929 A1 discloses a shoulder implant assembly including a frame member, a cup, and a glenosphere. The frame member can have a central hub and a first arm extending therefrom. The glenosphere can have an outer articulating surface that is configured to articulate with a second cup. The glenosphere can be selectively coupled to the frame member in the reverse shoulder configuration.
US 2016/324649 A1 discloses a glenoid implant including a base plate and an articular component. The base plate can include a body and a support structure extending from a distal surface of the body. The articular component can be configured to removably couple to the base plate. The articular component can include a recessed portion configured to at least partially receive the body of the base plate.

### SUMMARY OF THE PRESENT DISCLOSURE

The invention is defined in claim 1.

The foregoing and additional aspects and implementations of the present disclosure will be apparent to those of ordinary skill in the art in view of the detailed description of various embodiments and/or implementations, which is made with reference to the drawings, a brief description of which is provided next.

### BRIEF DESCRIPTION OF THE DRAWINGS

The foregoing and other advantages of the present disclosure will become apparent upon reading the following detailed description and upon reference to the drawings.
FIG. 1 is an exploded view of a glenoid implant in a first configuration according to some implementations of the present disclosure;
FIG. 2 is a front isometric perspective view of the glenoid implant of FIG. 1 surgically interfaced to a scapula;
FIG. 3 is a side isometric perspective view of the glenoid implant of FIG. 1;
FIG. 4 is a front view of the glenoid implant of FIG. 1 with a first, second and third cross-section;
FIG. 5 is a side view of the glenoid implant of FIG. 4 at the first cross-section;
FIG. 6 is a side view of the glenoid implant of FIG. 4 at the second cross-section;
FIG. 7 is a side view of the glenoid implant of FIG. 4 at the third cross-section;
FIG. 8 is a front view of the glenoid implant of FIG. 1 surgically interfaced to a scapula, with a fourth, and fifth cross-section;
FIG. 9 is a side view of the glenoid implant of FIG. 8 at the first cross-section;
FIG. 10 is a side view of the glenoid implant of FIG. 8 at the second cross-section;
FIG. 11 is a front isometric exploded view of a glenoid implant in a second configuration according to some implementations of the present disclosure;
FIG. 12 is a side isometric perspective view of the glenoid implant of FIG. 11 surgically interfaced to a scapula;
FIG. 13 is a side isometric perspective view of the glenoid implant of FIG. 11;
FIG. 14 is a front view of the glenoid implant of FIG. 11 with a cross-section;
FIG. 15 is a side view of the glenoid implant of FIG. 14 at the cross-section;
FIG. 16 is a rear isometric exploded view of the glenoid implant of FIG. 11;
FIG. 17 is a front isometric exploded view of a glenoid implant in a third configuration according to some implementations of the present disclosure;
FIG. 18 is a rear isometric exploded view of the glenoid implant of FIG. 17;
FIG. 19 is a cross-sectional side view of the glenoid implant of FIG. 17 surgically interfaced to a scapula;
FIG. 20 is a rear isometric exploded view of a glenoid implant in a fourth configuration according to some implementations of the present disclosure;
FIG. 21 is a front isometric exploded view of the glenoid implant of FIG. 20;
FIG. 22 is a first side view of the glenoid implant of FIG. 20;
FIG. 23 is a second side view of the glenoid implant of FIG. 20;
FIG. 24 is a rear isometric exploded view of a glenoid implant in a fifth configuration according to some implementations of the present disclosure;
FIG. 25 is a front isometric exploded view of the glenoid implant of FIG. 24;
FIG. 26 is a side isometric view of the glenoid implant of FIG. 24;
FIG. 27 is a cross-sectional side view of the glenoid implant of FIG. 24;
FIG. 28 is a front isometric exploded view of a glenoid implant in a sixth configuration according to some implementations of the present disclosure;
FIG. 29 is a rear isometric exploded view of the glenoid implant of FIG. 28;
FIG. 30 is a front isometric view of the glenoid implant of FIG. 28;
FIG. 31 is a rear isometric view of the glenoid implant of FIG. 28;
FIG. 32 is a cross-sectional isometric view of the glenoid implant of FIG. 28;
FIG. 33 is a side view of the glenoid implant of FIG. 28 in a first implementation;
FIG. 34 is a side view of the glenoid implant of FIG. 28 in a second implementation;
FIG. 35 is a side view of the glenoid implant of FIG. 28 in a third implementation;
FIG. 36 is a rear isometric exploded view of a glenoid implant in a seventh configuration according to some implementations of the present disclosure;
FIG. 37 is a front isometric exploded view of the glenoid implant of FIG. 36;
FIG. 38 is a front isometric view of a glenoid implant in an eighth configuration according to some implementations of the present disclosure;
FIG. 39 is a rear isometric view of the glenoid implant of FIG. 38;
FIG. 40 is a cross-sectional isometric view of the glenoid implant of FIG. 38;
FIG. 41 is a front isometric view of a glenoid implant in a ninth configuration according to some implementations of the present disclosure;
FIG. 42 is a rear isometric view of the glenoid implant of FIG. 41;
FIG. 43 is a cross-sectional isometric view of the glenoid implant of FIG. 41;
FIG. 44 is a front isometric exploded view of a glenoid implant in a tenth configuration according to some implementations of the present disclosure;
FIG. 45 is a rear isometric exploded view of the glenoid implant of FIG. 44;
FIG. 46 is a right rear isometric view of the glenoid implant of FIG. 44;
FIG. 47 is a left rear isometric view of the glenoid implant of FIG. 44;
FIG. 48 is a cross-sectional side view of the glenoid implant of FIG. 44;
FIG. 49 is a front isometric view of a partial assembled glenoid implant of FIG. 44;
FIG. 50 is a front isometric view of a partial assembled glenoid implant of FIG. 44;
FIG. 51 is a rear isometric view of a glenosphere in a first configuration according to some implementations of the present disclosure;
FIG. 52 is a rear isometric view of a glenosphere in a second configuration according to some implementations of the present disclosure;
FIG. 53 is a rear isometric view of a glenosphere in a third configuration according to some implementations of the present disclosure;
FIG. 54 is a rear isometric exploded view of a glenoid implant in an eleventh configuration according to some implementations of the present disclosure;
FIG. 55 is a front isometric exploded view of the glenoid implant of FIG. 54;
FIG. 56 is a front isometric exploded view of a glenoid implant in a twelfth configuration according to some implementations of the present disclosure;
FIG. 57 is a right rear isometric view of the glenoid implant of FIG. 56;
FIG. 58 is a cross-sectional side view of the glenoid implant of FIG. 56;
FIG. 59 is a front isometric exploded view of a glenoid implant in a thirteenth configuration according to some implementations of the present disclosure;
FIG. 60 is a rear isometric view of the glenoid implant of FIG. 59;
FIG. 61 is a front isometric exploded view of a glenoid implant in a fourteenth configuration according to some implementations of the present disclosure;
FIG. 62 is a rear isometric view of the glenoid implant of FIG. 61;
FIG. 63 is a front isometric exploded view of a glenoid implant in a fifteenth configuration according to some implementations of the present disclosure;
FIG. 64 is a rear isometric view of the glenoid implant of FIG. 63;
FIG. 65 is a front isometric exploded view of a glenoid implant in a sixteenth configuration according to some implementations of the present disclosure;
FIG. 66 is a rear isometric view of the glenoid implant of FIG. 65;
FIG. 67 is a front isometric exploded view of a glenoid implant in a seventeenth configuration according to some implementations of the present disclosure;
FIG. 68 is a rear isometric view of the glenoid implant of FIG. 67;
FIG. 69 is a front isometric exploded view of a glenoid implant in an eighteenth configuration according to some implementations of the present disclosure;
FIG. 70 is a rear isometric view of the glenoid implant of FIG. 69;
FIG. 71 is a front isometric exploded view of a glenoid implant in a nineteenth configuration according to some implementations of the present disclosure;
FIG. 72 is a rear isometric view of the glenoid implant of FIG. 71;
FIG. 73 is a front isometric exploded view of a glenoid implant in a twentieth configuration according to some implementations of the present disclosure;
FIG. 74 is a rear isometric view of the glenoid implant of FIG. 73;
FIG. 75 is a front isometric exploded view of a glenoid implant in a twenty-first configuration according to some implementations of the present disclosure;
FIG. 76 is a rear isometric view of the glenoid implant of FIG. 75;
FIG. 77 is a front isometric view of a glenoid implant in a twenty-second configuration according to some implementations of the present disclosure;
FIG. 78 is a top view of the glenoid implant of FIG. 77 with a cross-section;
FIG. 79 is a side view of the glenoid implant of FIG. 78 at the cross-section;
FIG. 80 is a first side view of the glenoid implant of FIG. 77 with a cross-section;
FIG. 81 is the first side view of the glenoid implant of FIG. 80 at the cross-section;
FIG. 82 is a second side view of the glenoid implant of FIG. 77 with a cross-section;
FIG. 83 is the second side view of the glenoid implant of FIG. 82 at the cross-section;
FIG. 84 is a top isometric view of a glenoid implant in a twenty-third configuration according to some implementations of the present disclosure;
FIG. 85 is a side view of the glenoid implant of FIG. 84 with a cross-section;
FIG. 86 is the side view of the glenoid implant of FIG. 85 at the cross-section;
FIG. 87 is a top view of the glenoid implant of FIG. 84 with a cross-section;
FIG. 88 is the side view of the glenoid implant of FIG. 87 at the cross-section;
FIG. 89 is a top isometric view of a glenoid implant in a twenty-fourth configuration according to some implementations of the present disclosure;
FIG. 90 is a side view of the glenoid implant of FIG. 89 with a cross-section;
FIG. 91 is the side view of the glenoid implant of FIG. 90 at the cross-section;
FIG. 92 is a top view of the glenoid implant of FIG. 89 with a cross-section;
FIG. 93 is the side view of the glenoid implant of FIG. 92 at the cross-section;
FIG. 94 is a rear isometric exploded view of a glenoid implant in a twenty-fifth configuration according to some implementations of the present disclosure;
FIG. 95 is a front isometric view of the glenoid implant of FIG. 94;
FIG. 96 is a rear isometric exploded view of a glenoid implant in a twenty-sixth configuration according to some implementations of the present disclosure;
FIG. 97 is a front isometric view of the glenoid implant of FIG. 96;
FIG. 98 is an exploded view of a glenoid implant in a twenty-seventh configuration according to some implementations of the present disclosure;
FIG. 99 is a front isometric perspective view of the glenoid implant of FIG. 98 surgically interfaced to a scapula;
FIG. 100 is an exploded view of a glenoid implant in a twenty-eight configuration according to some implementations of the present disclosure; and
FIG. 101 is a front isometric perspective view of the glenoid implant of FIG. 100 surgically interfaced to a scapula.

While the present disclosure is susceptible to various modifications and alternative forms, specific implementations and embodiments have been shown by way of example in the drawings and will be described in detail herein. It should be understood, however, that the present disclosure is not intended to be limited to the particular forms disclosed. Rather, the present invention is defined by the appended claims.

### DETAILED DESCRIPTION

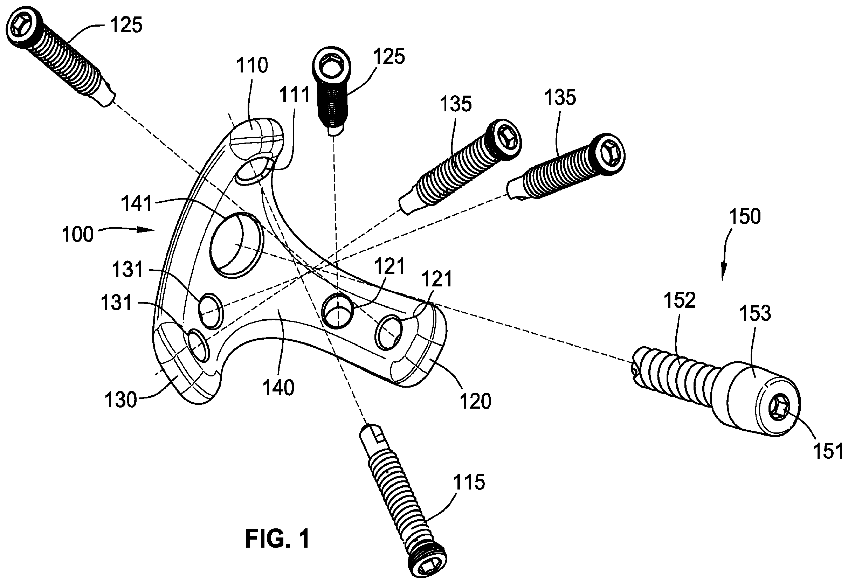

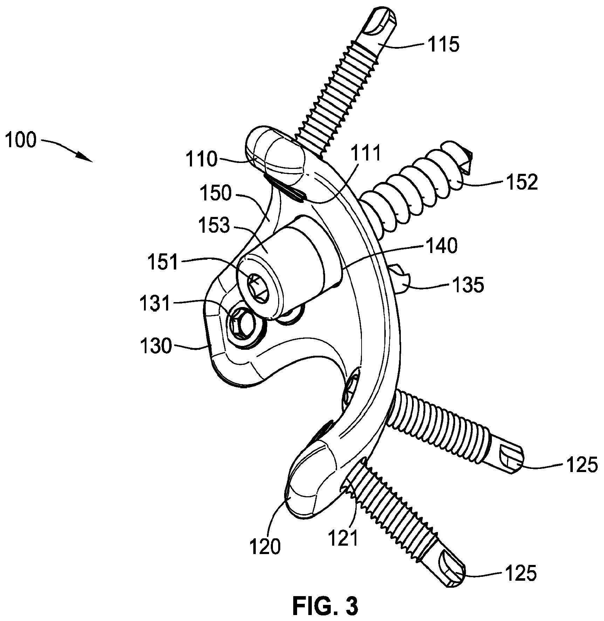

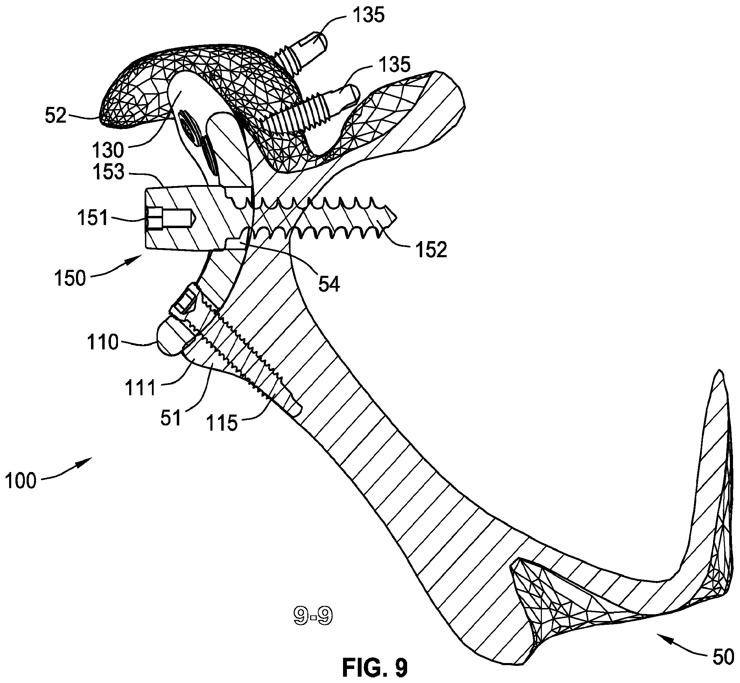

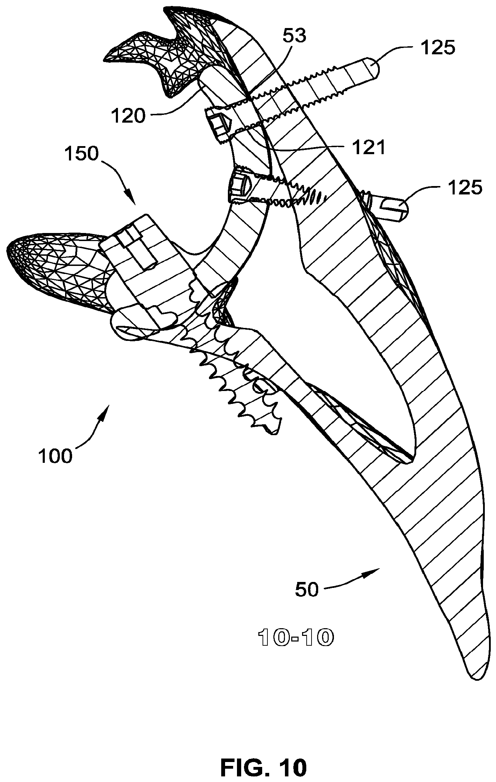

Referring generally to FIGS. 1-10, a glenoid implant 100 is illustrated. FIG. 1 is an exploded view of a glenoid implant 100. FIG. 3 is a side isometric perspective view of the glenoid implant 100. The glenoid implant 100 can be implemented in cases of severe bone loss and/or in cases of a fractured glenoid, where, for example, the glenoid implant 100 can be used to hold the fractured glenoid together so it can heal. The glenoid implant 100 includes a baseplate 140 and a first leg 110, a second leg 120, and a third leg 130 extending from the base plate 140. The first leg 110 extends from the baseplate 140 in a first direction, the second leg 120 extends from the baseplate 140 in a second direction, and the third leg 130 extends from the baseplate 140 in a third direction. The legs are curved with respect to the baseplate 140. In alternative implementations, the legs 110, 120, 130 can extend radially outward from the baseplate 140 without curvature.

The baseplate 140 includes a central aperture 141 therethrough. The first leg 110 also includes an aperture 111. The aperture 111 is configured to receive a portion of a first fastening element 115 therethrough. The second leg 120 includes an aperture 121. The aperture 121 is configured to receive a portion of a second fastening element 125 therethrough. Similarly, the third leg 130 includes an aperture 131. The aperture 131 is configured to receive a portion of a third fastening element 135 therethrough. While the first 110, second 120, and third 130 legs are described with one aperture, it should be understood that each leg can contain more than one aperture. In some implementations, the second leg 120 is longer than the first leg 110 and also longer than the third leg 130.

The glenoid implant 100 can also include a central fastening element 150 configured to be at least partially positioned through the central aperture 141 of the baseplate 140. The central fastening element 150 can be configured as a compression screw, or as a lock screw. The fastening element 150 includes a screw component or a threaded shaft 152, a head element 153, and a receiving element 151. The receiving element 151 is shaped to receive a tool for turning and/or installing the central fastening element 150. In some implementations, the head 153 of the central fastening element 150 is configured to be coupled with one or more additional components using a Morse taper press fit. In alternative implementations, the head 153 of the central fastening element 150 is configured to be coupled with a humeral head.

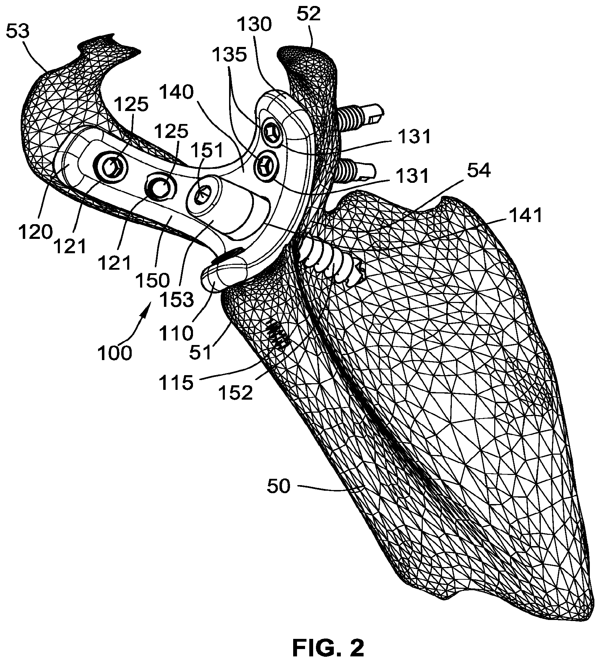

As best shown in FIG. 2, the glenoid implant 100 can be surgically interfaced and/or installed in a scapula 50 of a patient. For the purposes of this disclosure, the scapula 50 is illustrated with four portions. The first portion 51 of the scapula includes the Infraglenoid tubercle (also referred to as the lateral boarder of the scapula) and/or the For Subscapularis, the second portion 53 includes the Acromion, the third portion 52 includes the Coracoid, and the fourth portion 54 includes the glenoid cavity. The first fastening element 115 can aid in securing the first leg 110 to the Infraglenoid tubercle and/or the For Subscapularis. The second fastening element 125 can aid in securing the second leg 120 to the Acromion. The third fastening element 135 can aid in securing the third leg 130 to the Coracoid. The central fastening element 150 can aid in securing the baseplate 140 to the glenoid cavity. For example, the central fastening element 150 can compress the baseplate 140 to the glenoid cavity. In alternative implementations, the central fastening element 150 can simply attach to the baseplate 140, not pulling the baseplate 140 into the glenoid cavity.

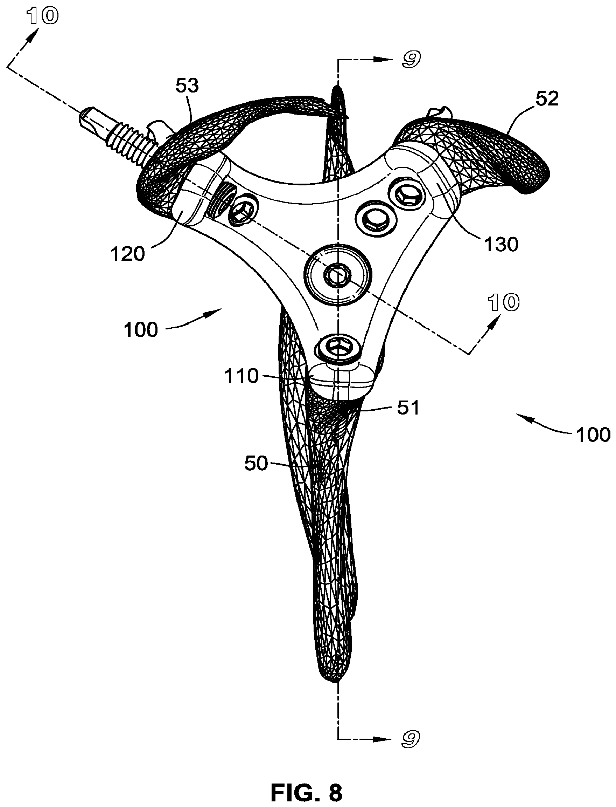

In some implementations, the first fastening element 115, the second fastening element 125, and the third fastening element 135 are configured to provide the glenoid implant 100 with bi-cortical support. In this way, the first fastening element 115, the second fastening element 125, and the third fastening element 135 are configured to protrude from the scapula 50 opposite the glenoid implant 100, to potentially connect two cortical plates. This is illustrated in FIGS. 8, 9 and 10. In some implementations, the first fastening element 115, the second fastening element 125, and the third fastening element 135 are configured to provide the glenoid implant 100 with uni-cortical support. In some implementations, the first fastening element 115, the second fastening element 125, and the third fastening element 135 can be self-tapping.

The first leg 110, the second leg 120, and the third leg 130 are curved to account for curvature of the Infraglenoid tubercle, the Acromion, and the Coracoid. In this way, a radius of curvature of the first leg 110, the second leg 120, and the third leg 130 aid in a substantial portion of the glenoid implant 100 directly abutting the Infraglenoid tubercle, the Acromion, and the Coracoid, respectively. In some implementations, at least seventy-five percent of a rear surface of the glenoid implant 100 directly abuts the scapula 50 prior to bone grafting material being applied. In some implementations, at least eighty percent, eighty-five percent, ninety percent, or ninety-five percent of a rear surface of the glenoid implant 100 directly abuts the scapula 50 prior to bone grafting material being applied.

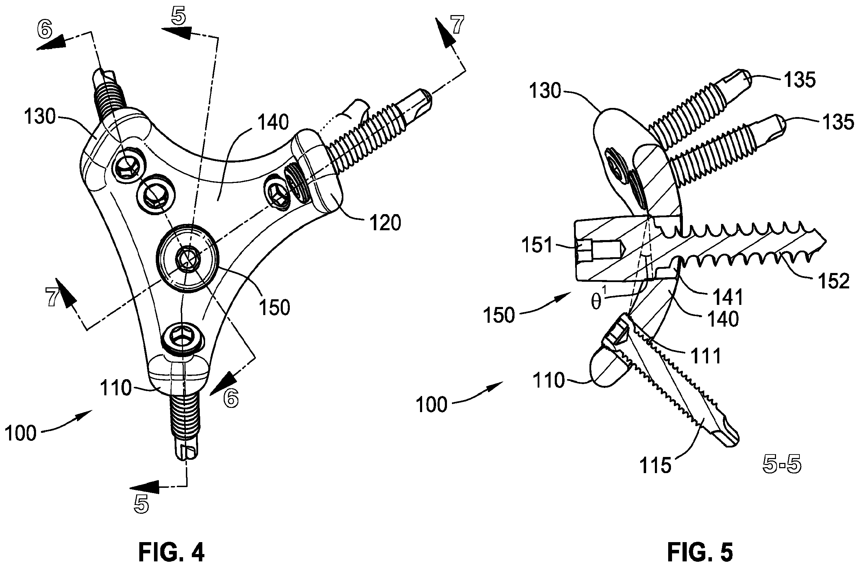

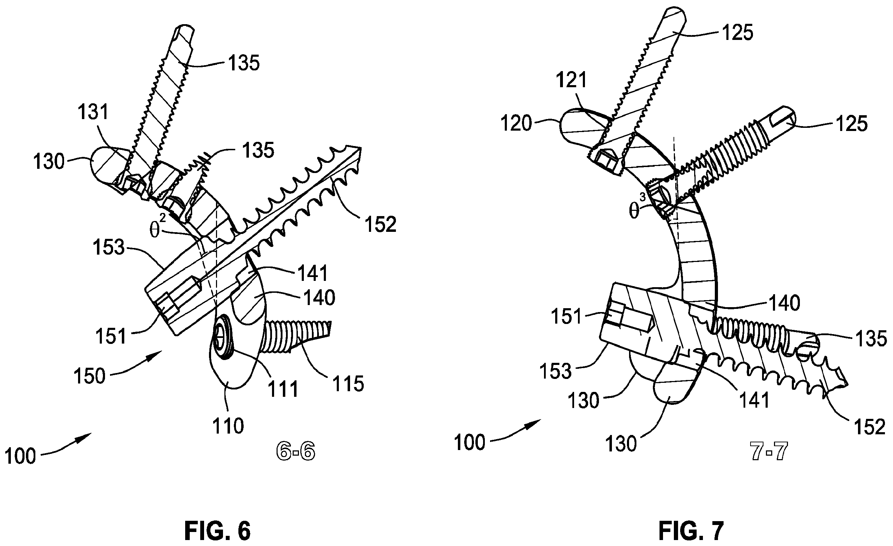

FIG. 4 is a front view of the glenoid implant 100 with a first cross-section 5-5, second cross-section 6-6, and third cross-section 7-7. FIG. 5 is a side cross-sectional view of the glenoid implant 100 at the first cross-section 5-5. FIG. 6 is a side cross-sectional view of the glenoid implant 100 at the second cross-section 6-6. FIG. 7 is a side cross-sectional view of the glenoid implant 100 at the third cross-section 7-7. As best shown in FIG. 5, the first leg 110 extends from the baseplate 140 generally along a first arc θ¹. As shown in FIG. 7, the second leg 120 extends from the baseplate 140 generally along a third arc θ³. As shown in FIG. 6, the third leg 130 can extend from the baseplate 140 generally along a second arc θ².

The radii of curvatures for each of the legs can vary with respect to one another. For example, the radius of curvature for the first arc θ¹ can be larger than the radius of curvature for the second arc θ². In some alternative implementations, the radius of curvature for the second arc θ² is larger than the radius of curvature for the first arc θ¹. Moreover, the radius of curvature for the first arc θ¹ can be the same as a radius of curvature for the second arc θ². This is the same with respect to the radius of curvature for the third arc θ³. In some implementations, the radius of curvature for the third arc θ³ is smaller than the radius of curvature for the second arc θ² and the radius of curvature for the first arc θ¹. In alternative embodiments, the radius of curvature for the third arc θ³ is the same as the radius of curvature for the second arc θ² and the radius of curvature for the first arc θ¹.

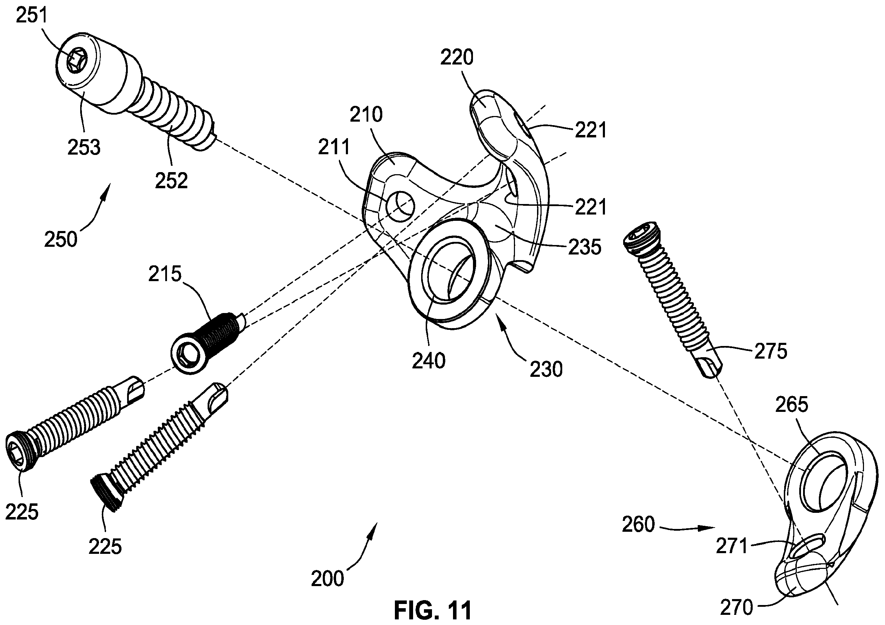

As illustrated herein, the baseplate 140, the first leg 110, the second leg 120, and the third leg 130 form a single monolithic part. However, in some implementations, the baseplate 140 can include a first portion coupled to a second portion such that the central aperture is defined by both the first portion and the second portion. In this case, the first portion of the baseplate, the first leg 110, and the second leg 120 form a first monolithic part and the second portion of the baseplate and the third leg 130 form a second monolithic part that is separate and distinct from the first monolithic part. This is discussed in greater detail with respect to FIG. 11.

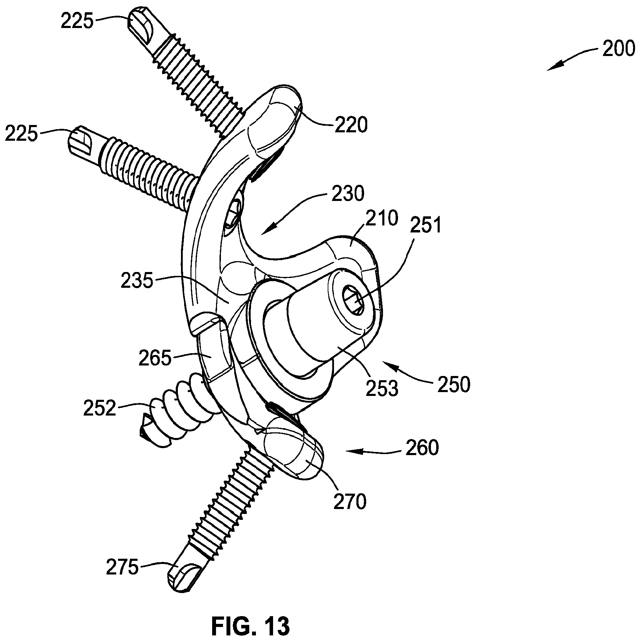

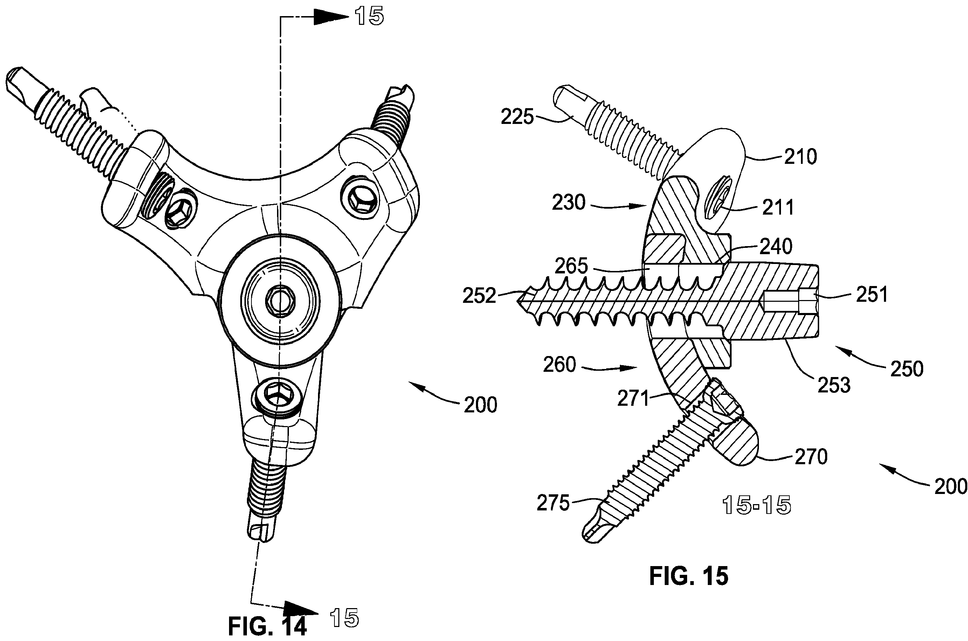

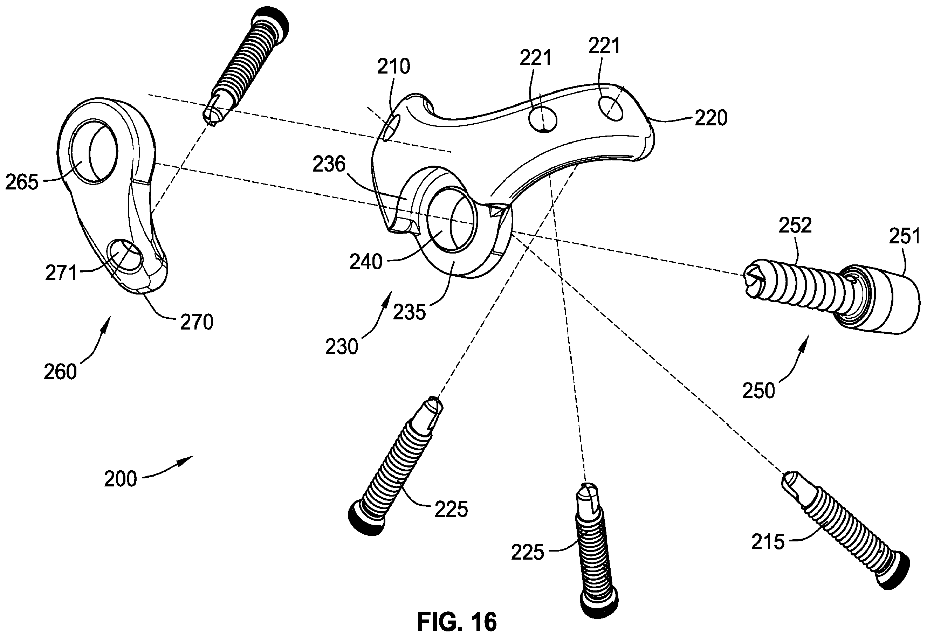

Now referring to FIGS. 11-16, a glenoid implant 200 is illustrated. FIG. 11 is a front isometric exploded view of the glenoid implant 200. FIG. 13 is a side isometric perspective view of the glenoid implant 200 in an assembled form. FIG. 14 is a front view of the glenoid implant 200 with a cross-section 15-15. FIG. 15 is a side cross-sectional view of the glenoid implant 200 at the cross-section 15-15. FIG. 16 is a rear isometric exploded view of the glenoid implant 200. The glenoid implant 200 includes a base with a first portion 230 and a second portion 260. The first portion 230 includes a first aperture 240 therethrough. The second portion 260 includes a second aperture 265 therethrough. The first aperture 240 can be configured to align with the second aperture 265, thereby defining a central aperture of the base, responsive to the first portion 230 being coupled to the second portion 260.

The first leg 210 extends from the first portion 230 of the base generally along a first arc. The second leg 220 extends from the first portion 230 of the base generally along a second arc. The first leg 210 can include a first aperture 211. The second leg 220 can include a second aperture 221. The first aperture 211 can be configured to receive a portion of a first fastening element 215 therethrough. Similarly, the second aperture 221 can be configured to receive a portion of a second fastening element 225 therethrough. The glenoid implant 200 can also include a central fastening element 250 configured to be at least partially positioned through the first aperture 240 and the aligned second aperture 265.

The adjustable third leg 270 extends from the second portion 260 of the base generally along a third arc in response to the first portion 230 being coupled to the second portion 260. The adjustable third leg 270 can include a third aperture 271 therein configured to receive a portion of a third fastening element 275 therethrough.

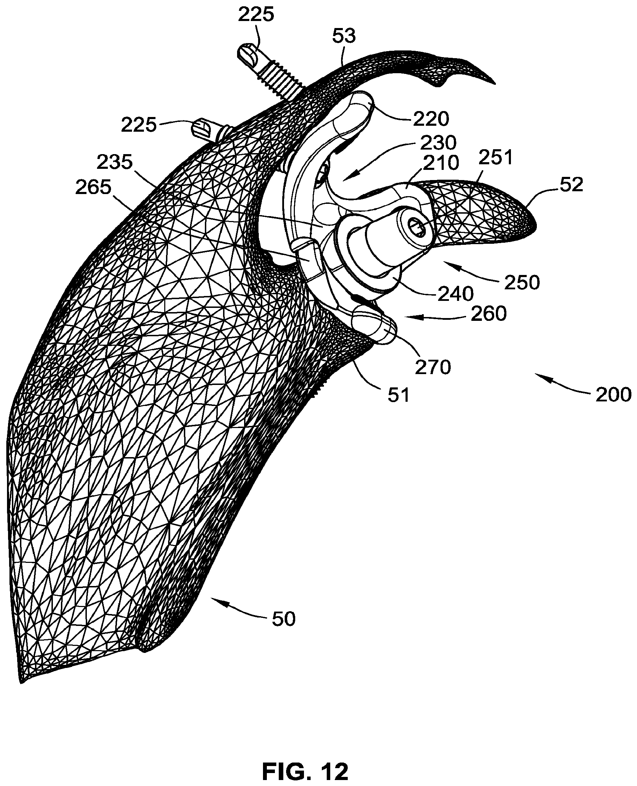

As best shown in FIG. 12, the glenoid implant 200 can be surgically interfaced and/or installed in the scapula 50. The first fastening element 215 (FIG. 11) can aid in securing the first leg 210 to the Coracoid. The second fastening element 225 (FIG. 11) can aid in securing the second leg 220 to the Acromion. The third fastening element 275 (FIG. 11) can aid in securing the adjustable third leg 270 to the Infraglenoid tubercle. The central fastening element 250 can aid in securing the base to the glenoid cavity. Similar to the glenoid implant 100, the first fastening element 215 and the second fastening element 225 are configured to protrude from the scapula 50 opposite the glenoid implant 200, to potentially connect two cortical plates. In alternative implementations, the first fastening element 215 and the second fastening element 225 are configured to provide the glenoid implant 100 with uni-cortical support.

The separation of the base into two components allows for the rotation of the second portion 260 about the first portion 230. In this way, the glenoid implant 200 can be adjusted for varying anatomies of the scapula 50. Specifically, an angle between the adjustable third leg 270 and the first leg 210 can be adjusted to account for a distance between the first portion of the scapula 52 and the third portion of the scapula 51. This is also the case with the angle between the adjustable third leg 270 and the second leg 220.

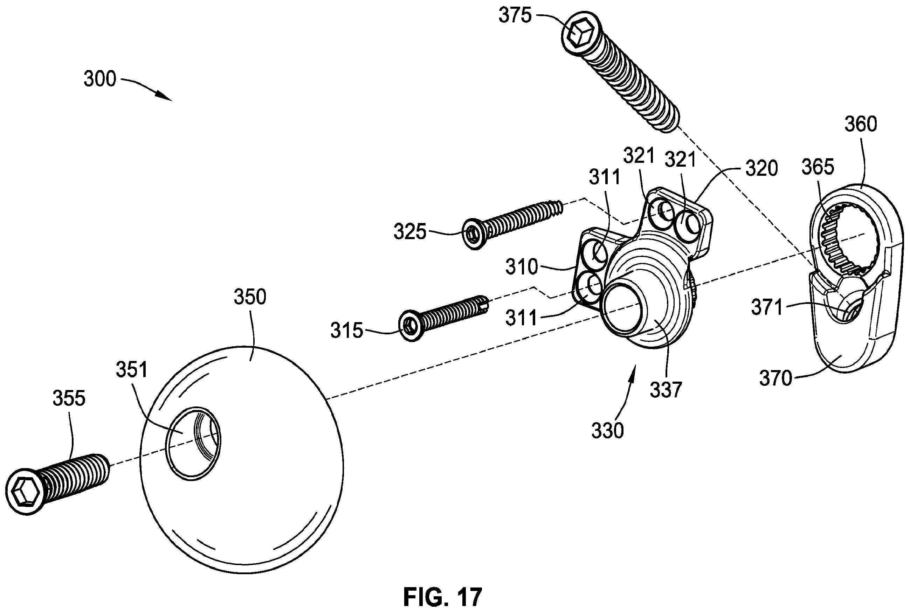

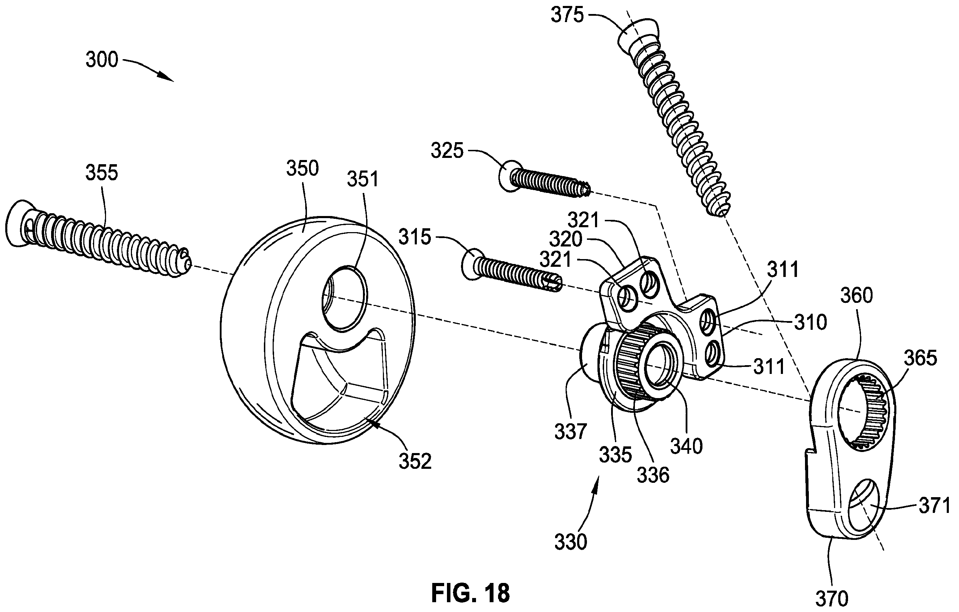

Referring generally to FIGS. 17-19, a glenoid implant 300 is illustrated. As shown in FIG. 17, a glenoid implant 300 can include a base with a first portion 330 and a second portion 360, which are similar to the first portion 230 and the second portion 260 of the base described above in connection with the glenoid implant 200. The first portion 330 can include a first trunnion 337 extending outward. The glenoid implant 300 can further include a glenosphere 350 with a central aperture 351 and a first receiving space 352 (shown in FIG. 18). The central aperture 351 of the glenosphere 350 can be coupled to the first trunnion 337 of the first portion 330. The first trunnion 337 can include the central aperture 340 therethrough. The glenosphere 350 can be configured to be secured to the glenoid implant 300 via a central fastening element 355 through the central aperture 351, the central aperture 340, and the central aperture 365.

FIG. 18 is a rear isometric exploded view of the glenoid implant 300. The rear of the first portion 330 can include a second trunnion 336 extending outward. The first trunnion 337 and the second trunnion 336 can include the central aperture 340 therethrough. The second trunnion 336 can include splines configured to rotationally couple with corresponding grooves in the second aperture 365 of the second portion 360. In alternative implementations, the second trunnion 336 can include grooves configured to rotationally couple with corresponding splines in the second aperture 365 of the second portion 360. The first receiving space 352 of the glenosphere 350 can be configured to receive an augment. This is described in further detail below.

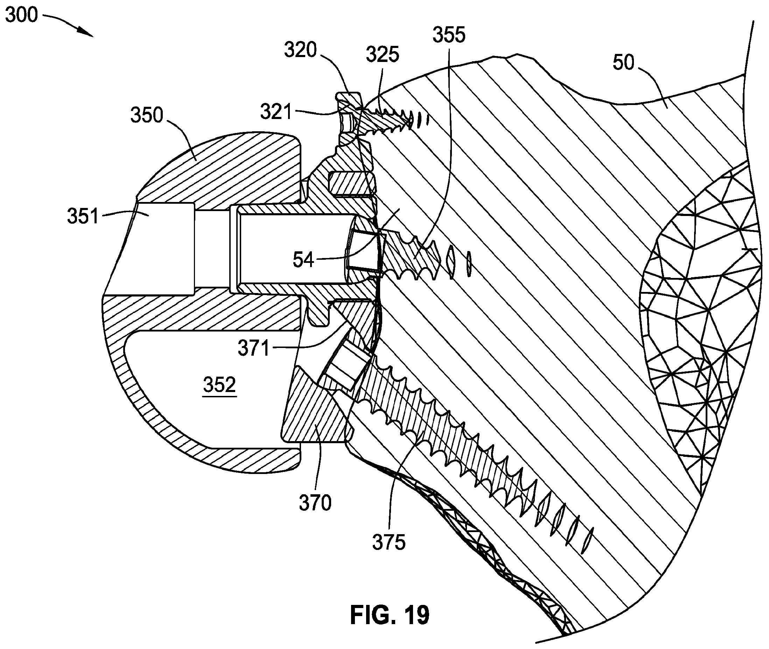

As shown in FIG. 19, the glenoid implant 300 can be surgically interfaced to a scapula 50 of a patient. The first fastening element 315 (not shown), the second fastening element 325, the third fastening element 375, and the central fastening element 355 can aid in securing the glenoid implant 300 into the glenoid cavity. The central fastening element 355 can compress the glenoid implant 300 to the glenoid cavity. In alternative implementations, the central fastening element 355 can attach to the glenoid implant 300 and not pull the glenoid implant 300 into the glenoid cavity.

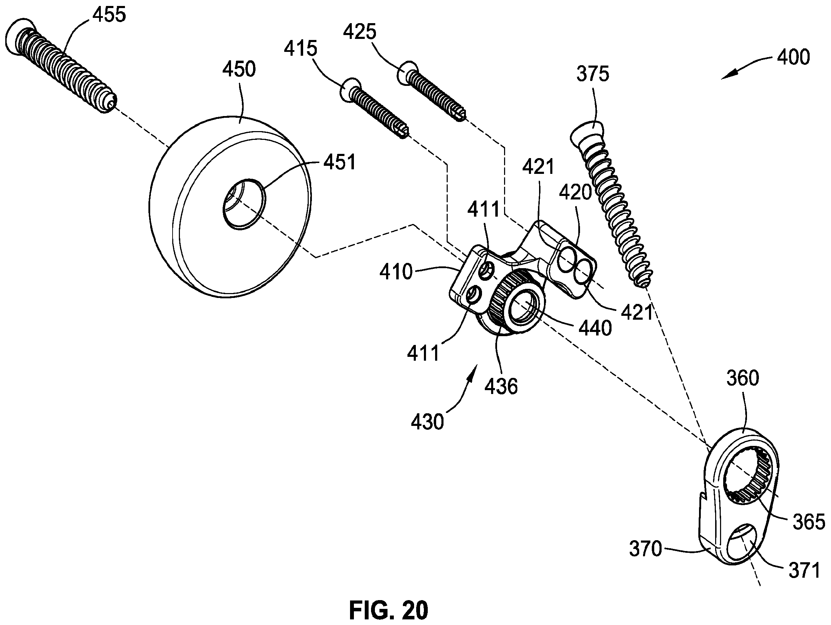

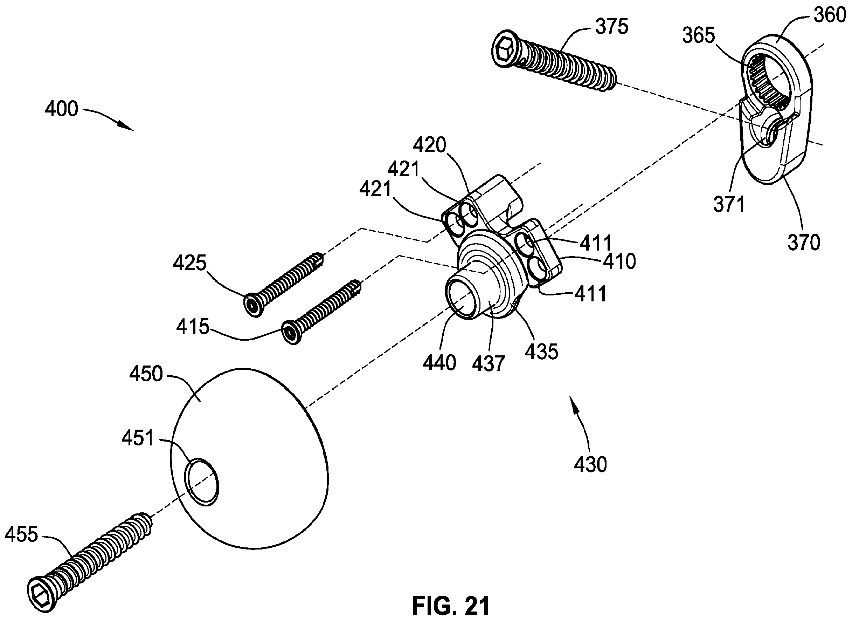

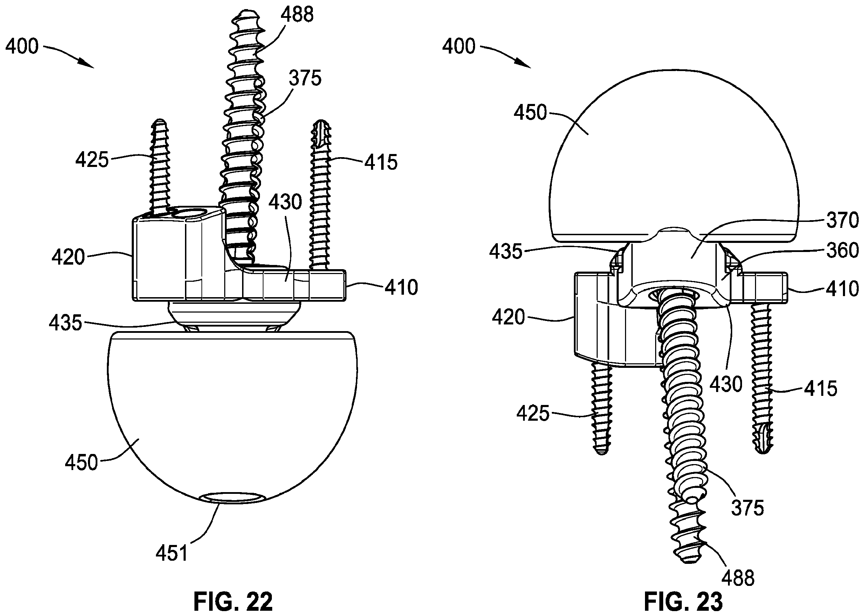

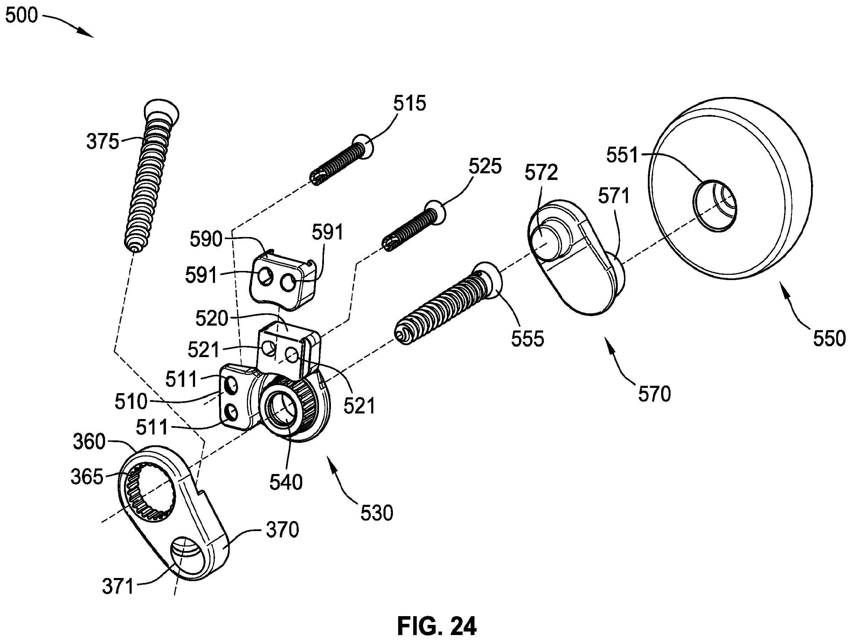

Referring generally to FIGS. 20-23, a glenoid implant 400 is illustrated. FIGS. 20 and 21 are front and rear isometric exploded views of the glenoid implant 400. FIGS. 22 and 23 are first side and second side views of the assembled glenoid implant 400. The glenoid implant 400 can include a first portion 430 and the second portion 360, which are the same as, or similar to, the first portion 330 and the second portion 360 of the base described above in connection with the glenoid implant 300. Specific to the glenoid implant 400, the second leg 420 of the glenoid implant 400 can have a different geometry than the first leg 410. Specifically, the second leg 420 can be thicker than the first leg 410. In some implementations, the varying thickness of the legs can be fixed. In other implementations, the thickness of the legs can be adjusted during the surgical procedure. This is illustrated in the glenoid implant 500 of FIG. 24.

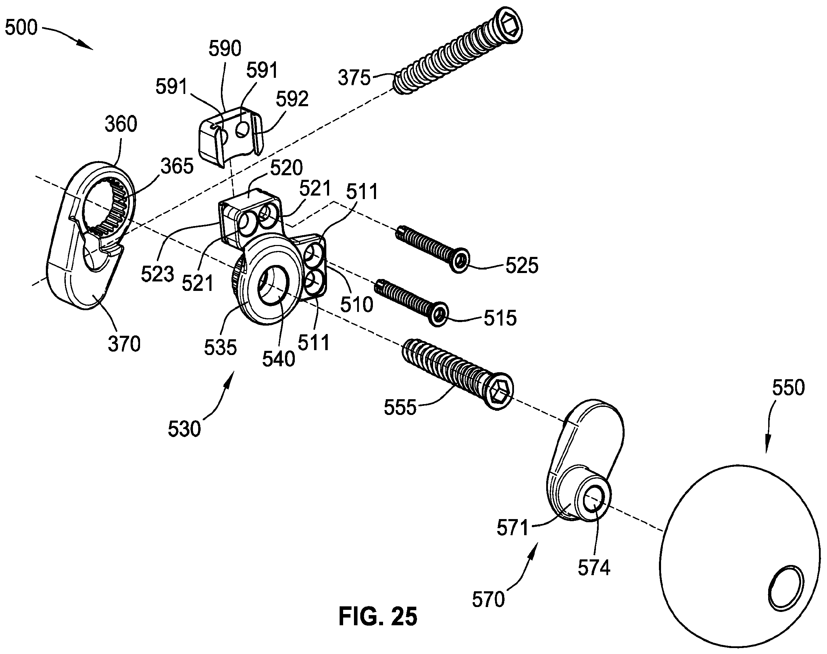

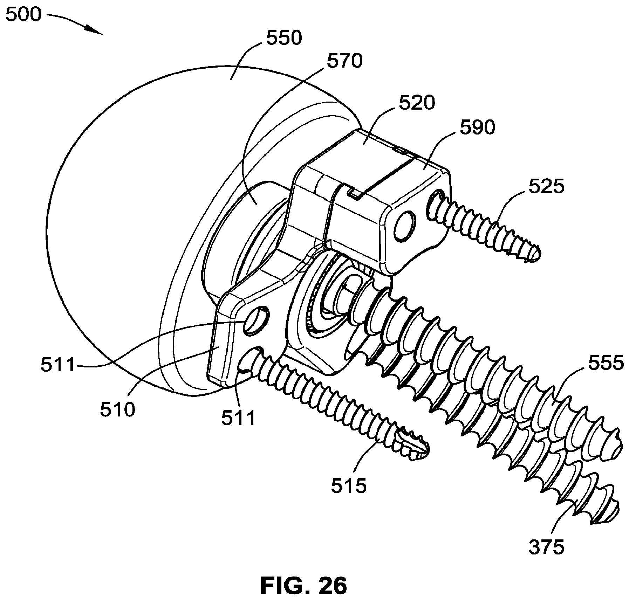

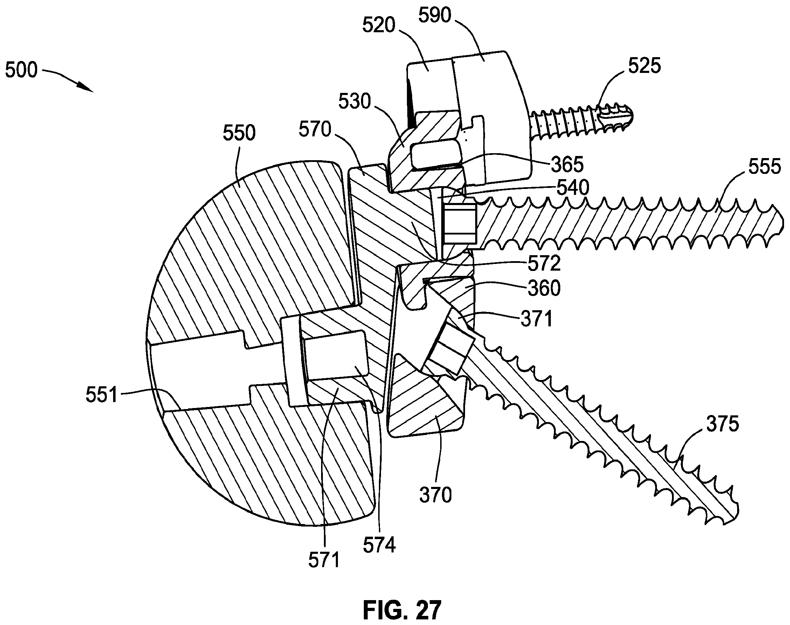

Referring generally to FIGS. 24-27, a glenoid implant 500 is illustrated. FIGS. 24 and 25 are front and rear isometric exploded views of the glenoid implant 500. FIG. 26 is a rear side isometric view of the glenoid implant 500. FIG. 27 is a cross-sectional side view of the glenoid implant 500. The glenoid implant 500 can include a first portion 530 and the second portion 360, which are the same as, or similar to, the first portion 430 and the second portion 360 of the base described above in connection with the glenoid implant 400.

Specific to the glenoid implant 500, the second leg 520 can be configured to be coupled to an augment 590, to increase the thickness of the second leg 520. Specifically, the augment 590 can include a slot 592 configured to receive a projection 523 extending from the second leg 520, such that the augment 590 slides onto and is coupled to the second leg 520. The augment 590 can include at least one aperture 591. The aperture 591 can be aligned with the aperture 521 of the second leg 521. In this way, the second fastening element 525 can be received through the second aperture 521 of the second leg 520 and the augment aperture 591 to aid in coupling the second leg 520 and the augment 590 to the glenoid cavity (similar to the glenoid cavity 300 of FIG. 19). While the glenoid implant 500 illustrates the augment 590 coupled to the second leg 520, it should be understood that the augment 590 can be coupled to either the first 510, second 520 or adjustable third leg 370.

The glenoid implant 500 can also include an offset element 570. The offset element 570 can include a base with a first trunnion 572 protruding from a first surface, and a second trunnion 571 protruding from a second surface opposite the first surface. The first trunnion 572 and the second trunnion 571 can have two distinct central axes. The first trunnion 572 can be aligned with the central aperture 540 of the first portion 530 and the central aperture 365 of the second portion 360. The second trunnion 571 can be aligned with the glenosphere central aperture 551. In this way, the glenosphere is coupled to the base such that a central axis of the central aperture 540 of the base is not aligned with a central axis of the aperture 551 within the glenosphere 550.

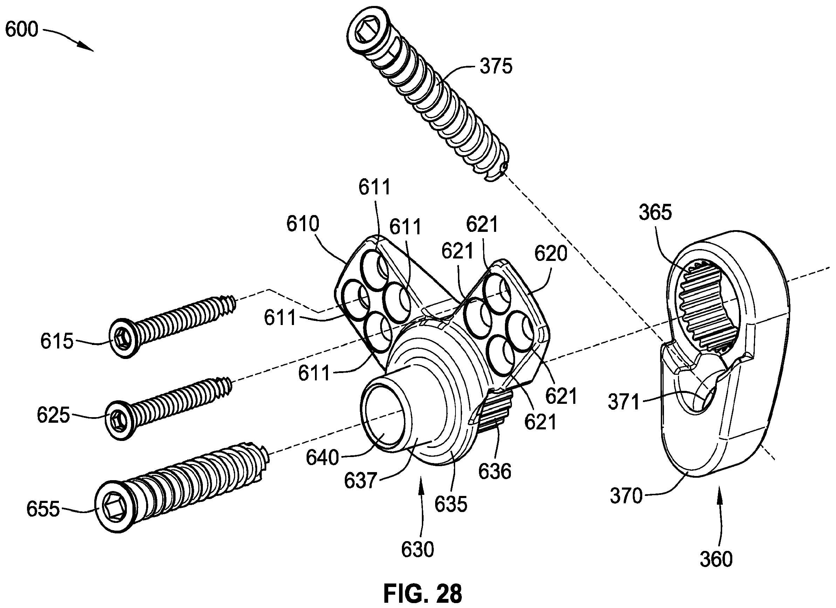

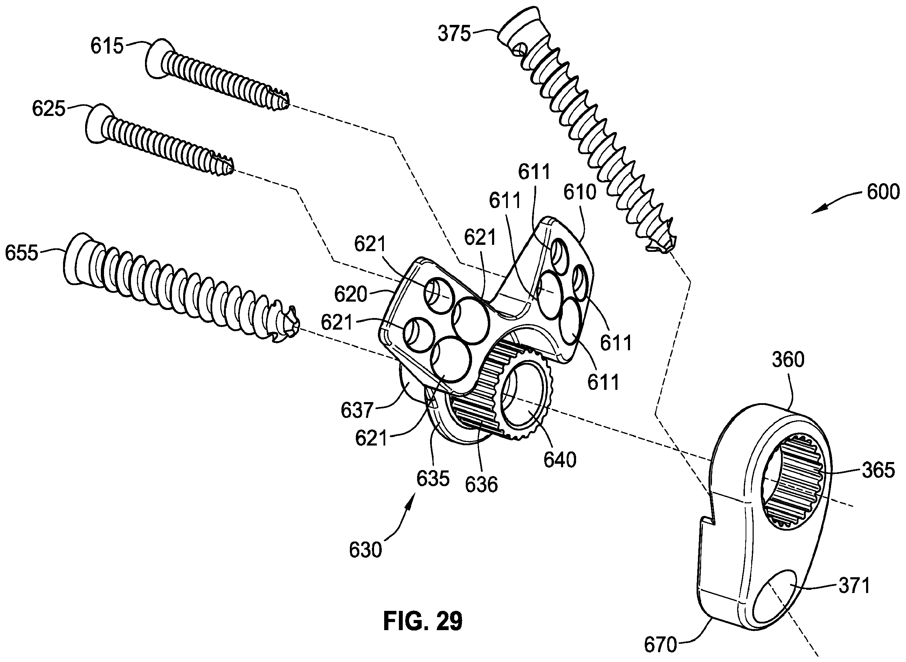

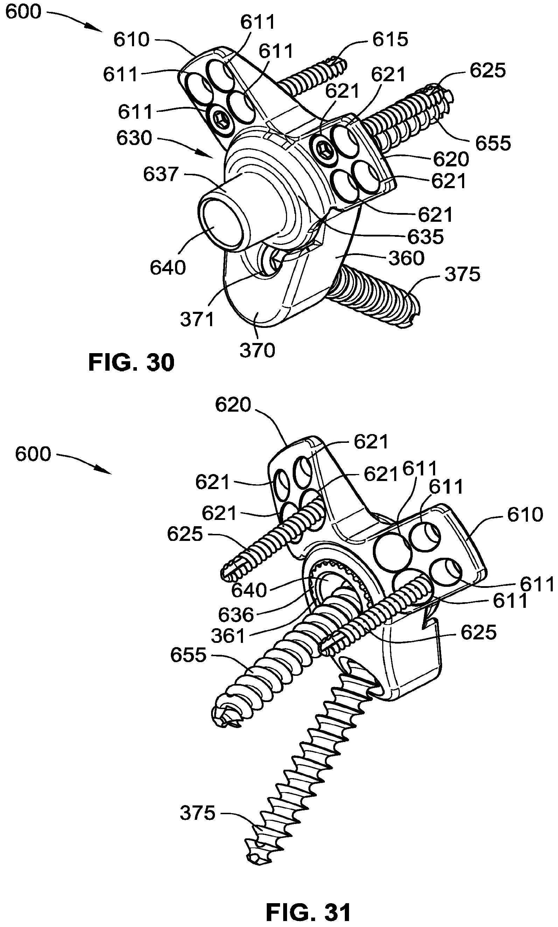

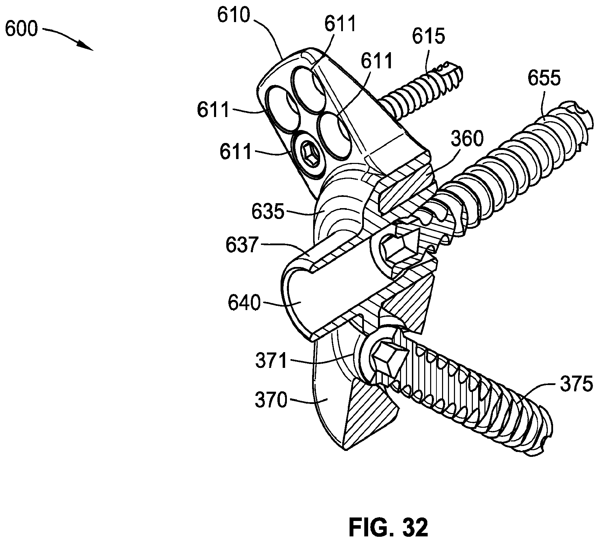

Referring generally to FIGS. 28-35, a glenoid implant 600 is illustrated. FIGS. 28 and 29 are front and rear isometric exploded views of the glenoid implant 600. FIGS. 30 and 31 are front and rear isometric views of the glenoid implant 600. FIG. 32 is a cross-sectional isometric view of the glenoid implant 600. The glenoid implant 600 can include a first portion 630 and the second portion 360, which are the same as, or similar to, the first portion 530 and the second portion 360 of the base described above in connection with the glenoid implant 500.

Specific to the glenoid implant 600, the first and second legs 610, 620 can be configured with additional apertures. As illustrated herein, the first leg 610 can include four apertures 611. Each aperture 611 can be configured to receive a portion of a first fastening element 615 therethrough. The second leg 620 can also include four apertures 621. Each aperture 621 can be configured to receive a portion of a second fastening element 625 therethrough. The additional apertures in the first and second legs 610, 620 assist with the lateralization of the glenoid implant 600.

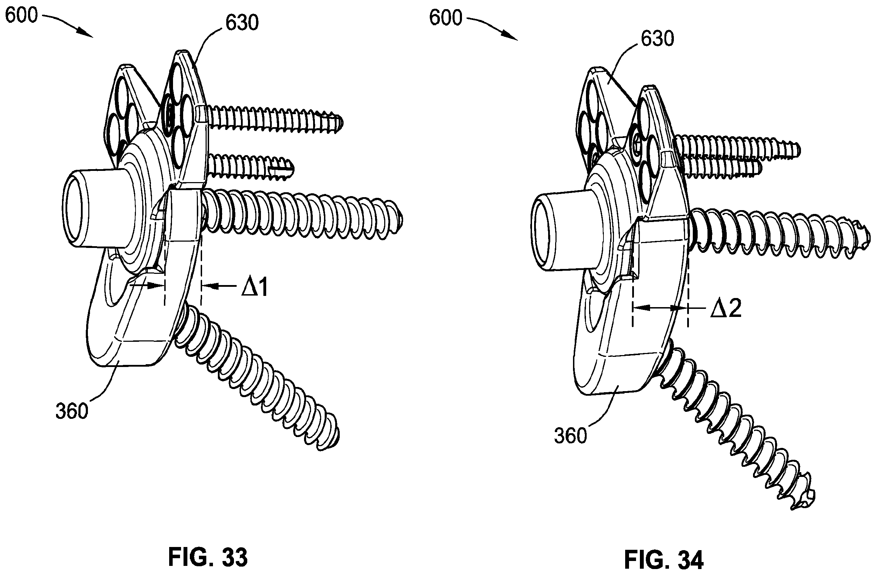

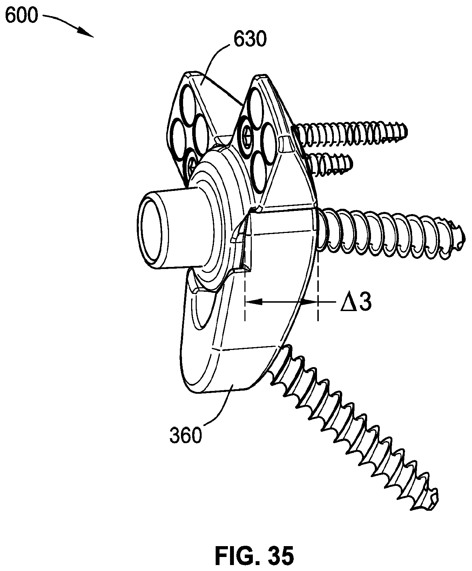

While the second component 360 has been illustrated with multiple implementations discussed above, it should be understood that the geometry of the second component 360 can also vary. FIGS. 33, 34 and 35 are side views of the glenoid implant 600 in a first, second, and third implementation, respectively. As best shown in FIG. 33, the second component 360 can include a first thickness Δ1. The first and second legs 610, 620 can also have a first thickness to account for the first thickness Δ1 of the second component 360. FIG. 34 illustrates the second component 360 with a second thickness Δ2. The second thickness Δ2 can include a 5 mm offset from the first thickness Δ1. The first and second legs 610, 620 can have a second thickness to account for the second thickness Δ2 of the second component 360. FIG. 35 illustrates the second component 360 with a third thickness Δ3. The third thickness Δ3 can include a 10 mm offset from the first thickness Δ1. The first and second legs 610, 620 can also have a third thickness to account for the third thickness Δ3 of the second component 360. In this way, once the first component 630 and the second component 360 is assembled, the rear of the glenoid implant 600 can have a continuous surface.

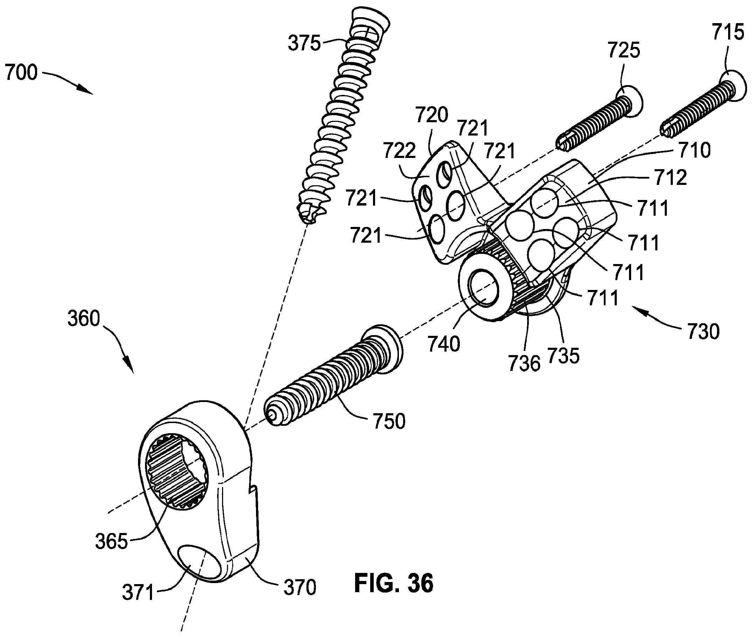

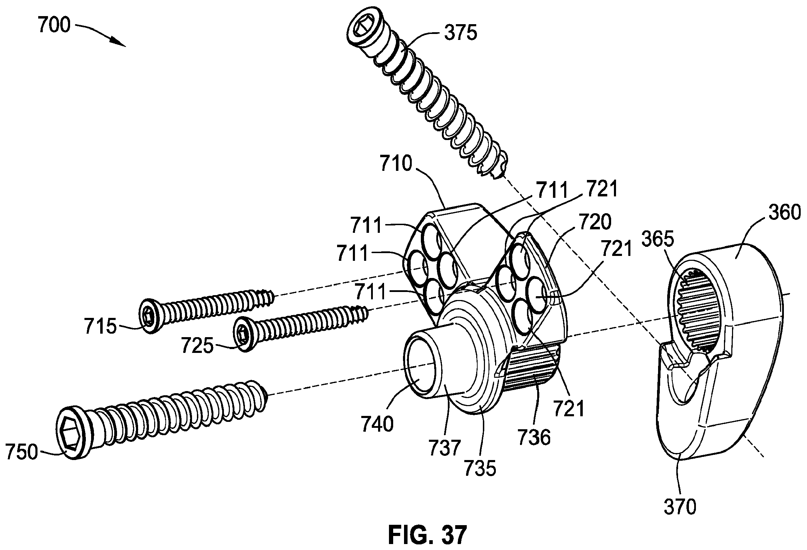

Referring generally to FIGS. 36-37, a glenoid implant 700 is illustrated. FIGS. 36 and 37 are front and rear isometric exploded views of the glenoid implant 700. The glenoid implant 700 can include a first portion 730 and the second portion 360, which are the same as, or similar to, the first portion 630 and the second portion 360 of the base described above in connection with the glenoid implant 600.

Specific to the glenoid implant 700, a scapula contacting surface 712 of the first leg 710 can have a different geometry than the scapula contacting surface 722 of the second leg 720. The scapula contacting surface 712 of the first leg 710 can include a convex surface while the scapula contacting surface 722 of the second leg 720, and the adjustable third leg 370 includes a concave surface. Moreover, the thickness of each leg can vary, as discussed above. While the scapula contacting surface 712 of the first leg 710 is illustrated with the convex surface, it should be understood that the contacting surface 722 of the second leg 720 or the adjustable third leg 360 can also have a convex surface.

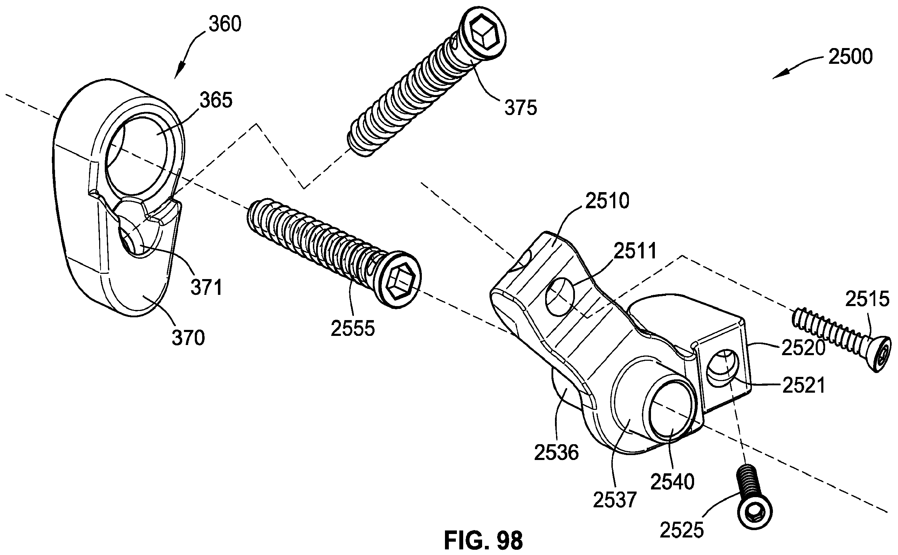

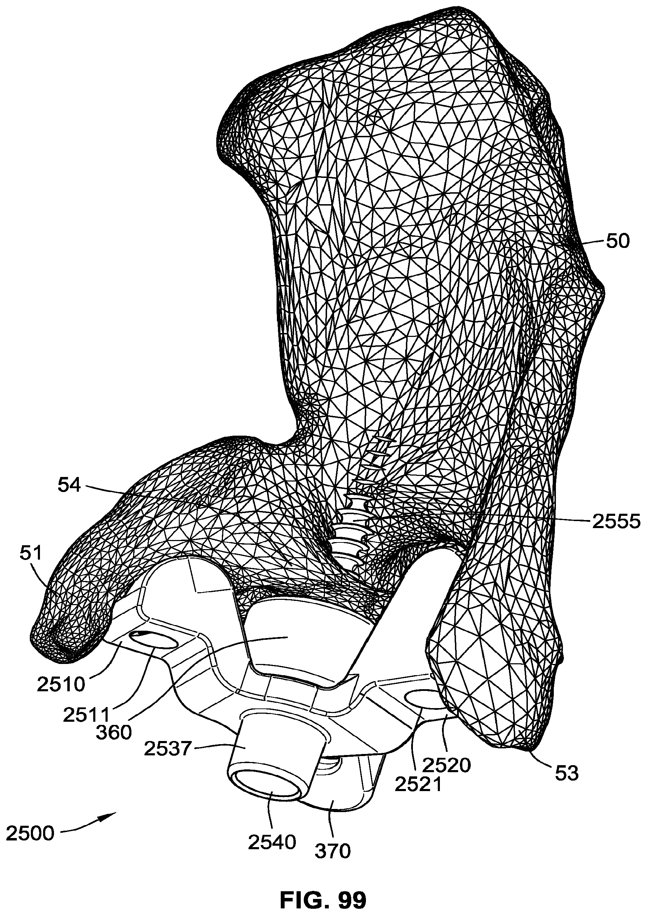

Referring momentarily to FIGS. 98-99, a glenoid implant 2500 is illustrated. FIGS. 98 is an exploded view of the glenoid implant 2500. FIG. 99 is a side isometric perspective view of the glenoid implant 2500. The glenoid implant 2500 can include a first portion 2530 and the second portion 360, which are the same as, or similar to, the first portion 730 and the second portion 360 of the base described above in connection with the glenoid implant 700.

Specific to the glenoid implant 2500, the first leg 2510 and the second leg 2520 can have a flat face surface and rounded rear surface configured to contact the scapula. In this configuration, each leg of the glenoid implant can be designed based on the profile of the scapula.

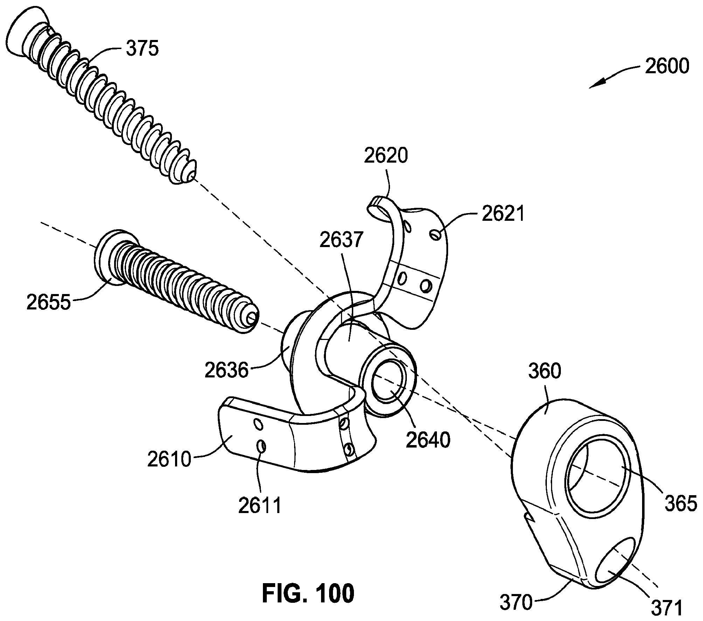

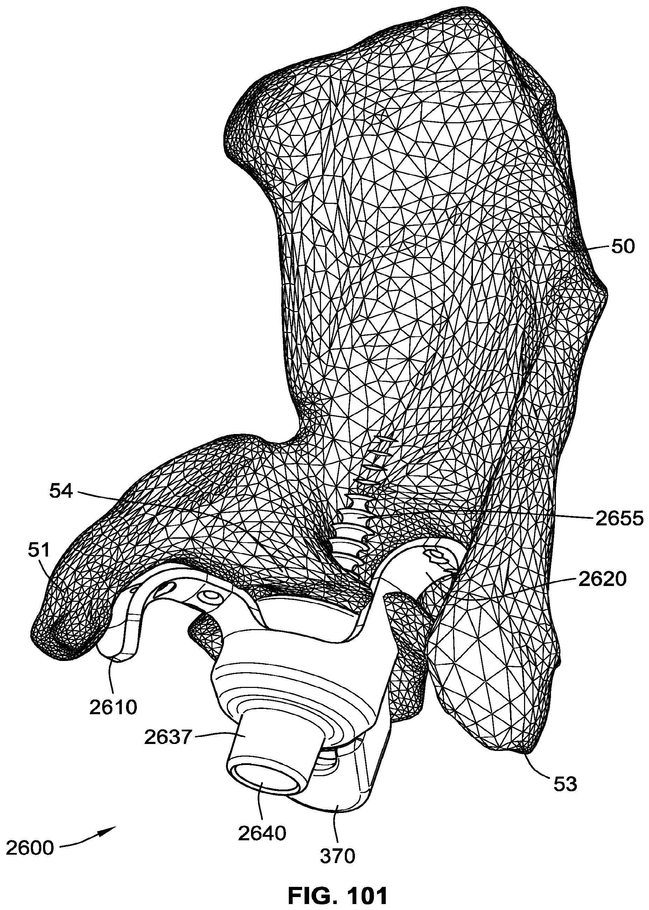

Referring momentarily to FIGS. 100-101, a glenoid implant 2600 is illustrated. FIGS. 100 is an exploded view of the glenoid implant 2600. FIG. 101 is a side isometric perspective view of the glenoid implant 2600. The glenoid implant 2600 can include a first portion 2630 and the second portion 360, which are the same as, or similar to, the first portion 2530 and the second portion 360 of the base described above in connection with the glenoid implant 2500.

Specific to the glenoid implant 2500, the first leg 2510 and the second leg 2520 can each have a unique arc. In this configuration, each leg of the glenoid implant can be designed based on the profile of the scapula. Specifically, the first leg 2510 and the second leg 2520 are bent in place depending on the anatomy of the scapula.

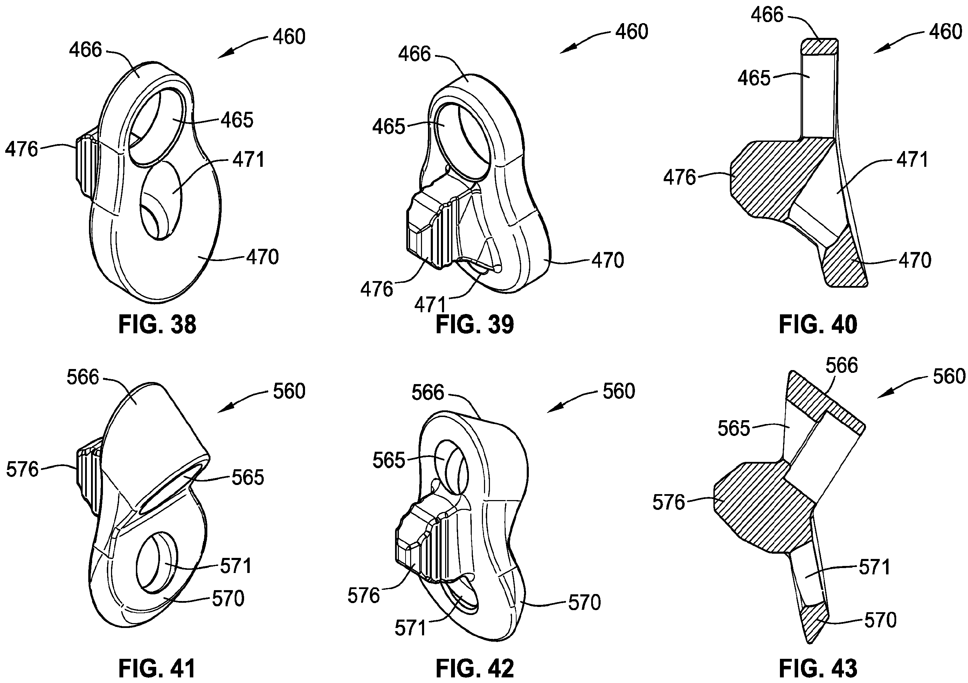

Referring generally to FIGS. 38-40, a second portion 460 is illustrated. FIGS. 38 and 39 are front and rear isometric views of the second portion 460. FIG. 40 is a cross-sectional isometric view of the second portion 460. The second portion 460 can include an adjustable leg 470 with an aperture 471 and a body 466. The adjustable leg 470 can be configured to extend from the body 466. The body 466 can include a central aperture 465. The second portion 460 can also include a keel component 476 extending from a rear surface of the second portion 460. The keel component 476 can be configured to be inserted into a cavity defect of the glenoid cavity of a scapula. This is discussed in greater detail below.

Referring generally to FIGS. 41-43, a second portion 560 is illustrated. FIGS. 41 and 42 are front and rear isometric views of the second portion 560. FIG. 43 is a cross-sectional isometric view of the second portion 560. The second portion 560 can include an adjustable leg 570 and a body 566, which are similar to, the adjustable leg 470 and the body 466 of the second portion 460 described above.

Specific to the second portion 560, the adjustable leg 570 can have a wider geometry than the adjustable leg 470 of the second portion 460. In addition, the body 566 can have a greater thickness than the body 466 of the second portion 460. It should be understood the second portion can vary in size and geometry based on the amount of bone loss of the patient. As best shown in FIGS. 40 and 43, the second portion 560 can be angled more for more severe bone loss. For example, the body 466 and the adjustable third leg 470 can be spaced between 130-135 degrees. Whereas, the body 566 and the adjustable third leg 570 can be spaced about 45 degrees. A glenosphere can be coupled to the body 466 or 566. This is discussed in greater detail below.

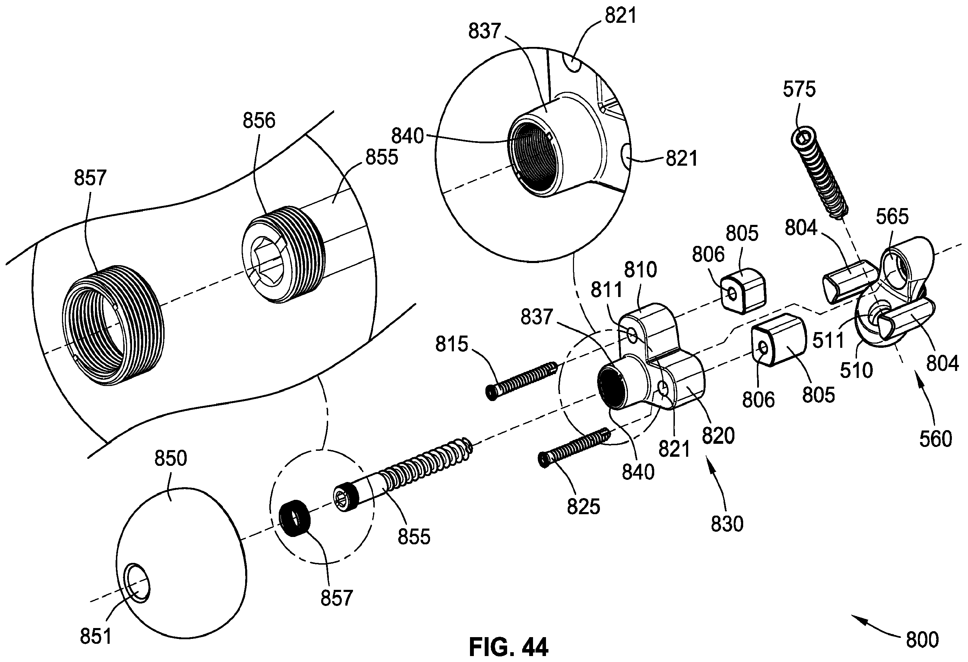

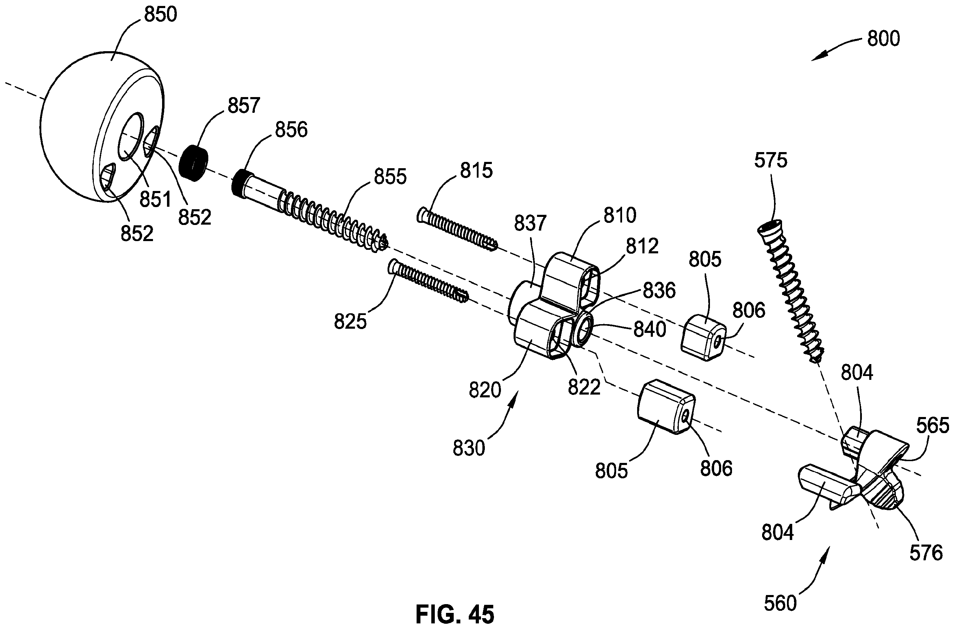

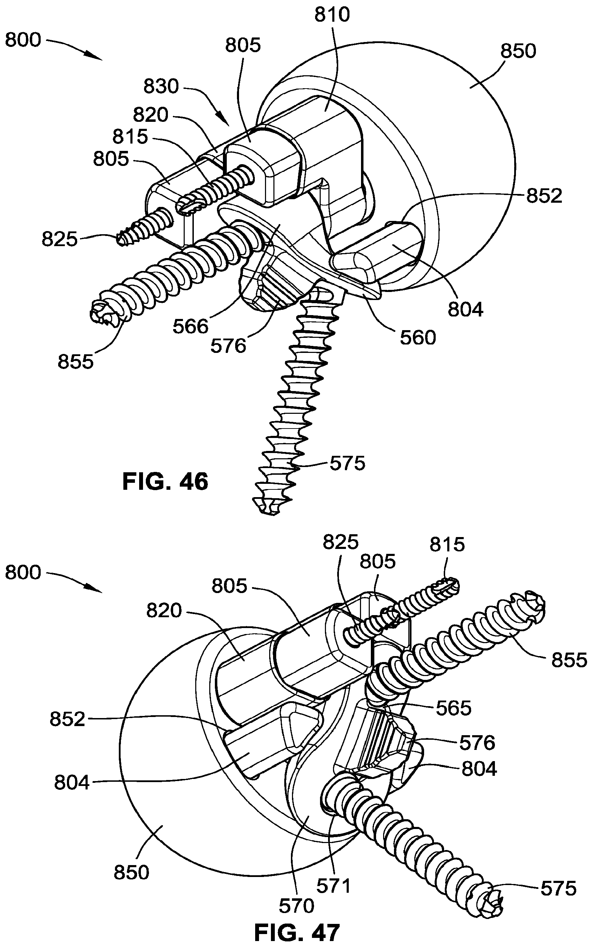

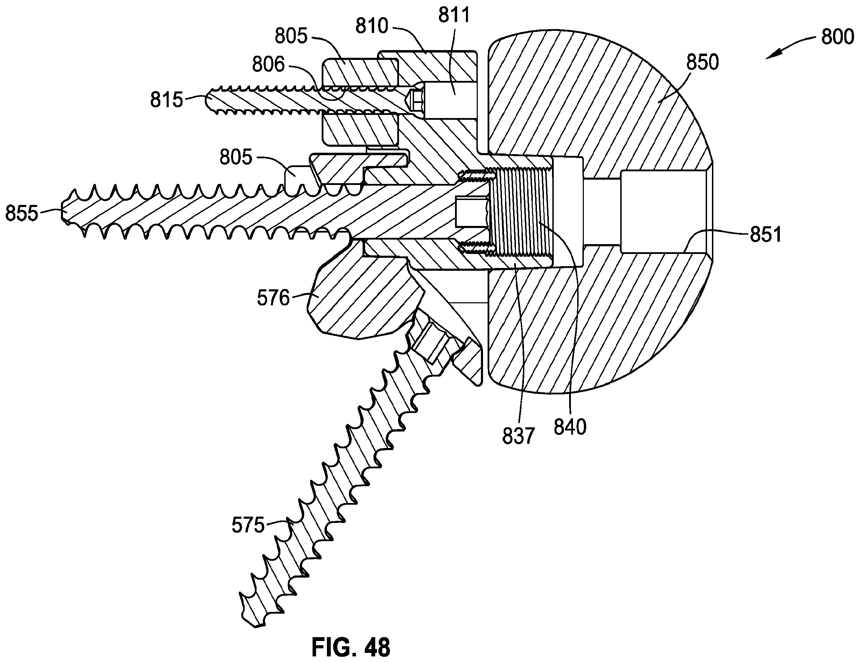

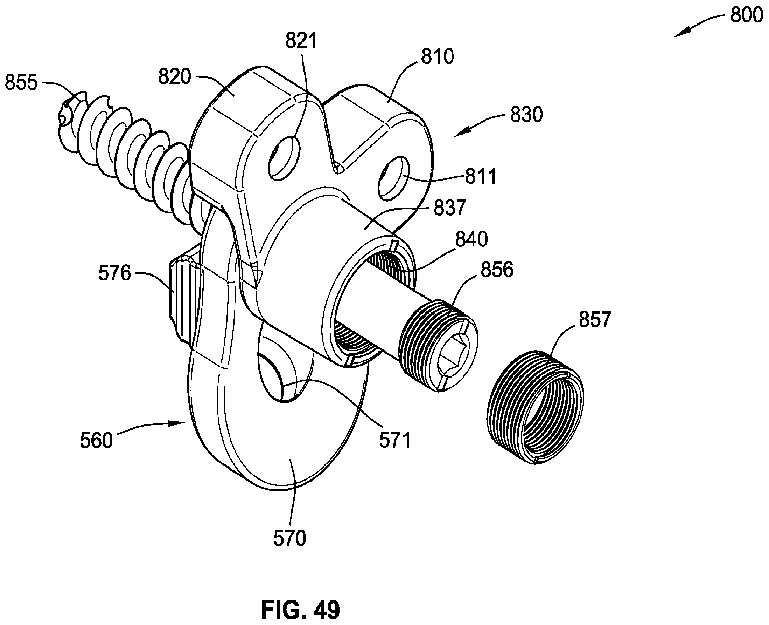

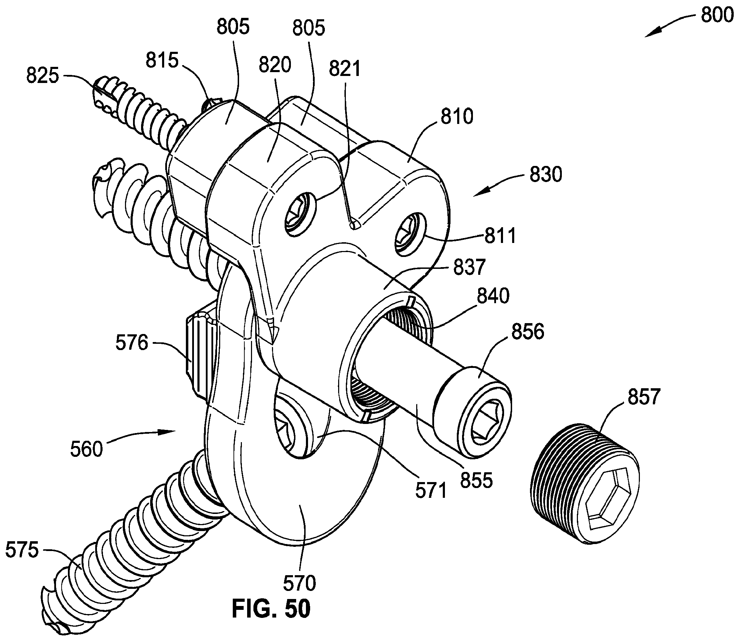

Referring generally to FIGS. 44-50, a glenoid implant 800 is illustrated. FIGS. 44 and 45 are front and rear isometric exploded views of the glenoid implant 800. FIGS. 46 and 47 are right and left rear isometric views of the glenoid implant 800. FIG. 48 is a cross-sectional side view of the glenoid implant 800. FIGS. 49 and 50 are front isometric views of a partial assembled glenoid implant 800.

The glenoid implant 800 can include a first portion 830 and the second portion 560, which are similar to the first portion 730 and the second portion 360 of the base described above in connection with the glenoid implant 700. Specific to the glenoid implant 800, the first and second legs 810, 820 can include a different geometry. The first leg 810 can have a single aperture 811, and the second leg 820 can have a single aperture 812. Furthermore, the first and second legs 810, 820 do not extend far from the first trunnion 837. In fact, the first portion 830 is smaller in size with respect to previous implementations discussed herein. The first trunnion 837 can include a central aperture 840 configured to align with the central aperture 565 of the second portion 560.

As best shown in FIG. 45, the first leg 810 can have a receiving space 812 configured to receive an augment 805. Similarly, the second leg 820 can have a receiving space 822 configured to receive an augment. The rear of the first portion 830 can also include a second trunnion 836 configured to be received within the central aperture 565 of the second portion 560. The augments 805 can be provided to provide additional support to the glenoid implant 800 by increasing the relative thickness of the leg. Each augment 805 can include a corresponding aperture 806 that aligns with, for example, the first aperture 811 in the first leg 810. The alignment of the apertures allows the first fastening element 815 to be received through the first aperture 811 of the first leg 810 and the aperture 806 of the augment 805.

The glenoid implant 800 can also include a central fastening element 855 configured to be at least partially positioned through the central aperture 840 of the first portion 830 and the central aperture 565 of the second portion 560. The glenoid implant 800 can also include a glenosphere 850 configured to be secured to the first and second portions 830, 560 via the central fastening element 855. As best shown in FIGS. 46 and 47, the glenosphere 850 can include receiving spaces 852 configured to receive an augment 804. The augments 804 in the bottom of the glenosphere 850 can be attached with a screw. Alternatively, the augments 804 can be tapered and hammered into the glenosphere 850.

As best shown in FIG. 49, the head 856 of the central fastening element 855, in some implementations, is threaded. The threaded head 856 can be configured to couple with a threaded element 857. The threaded element 857 is configured to be received within the central aperture 840 of the first portion 830, which is also threaded to receive the threaded element 857 in a threaded engagement. The central fastening element 855 and the threaded element 857 are configured to screw into place in alternative directions. That is, in some implementations, the central fastening element 855 is screwed into place in a first rotational direction (e.g., clockwise) and the threaded element 857 is screwed into place in a second opposing rotational direction (e.g., counterclockwise). In an alternative implementation shown in FIG. 50, the head 856 of the central fastening element 855 is not threaded. The non-threaded head 856 is configured to be couple with a different version of the threaded element 857. In this implementation shown in FIG. 50, the central fastening element 855 is installed normally as described above (e.g., threaded into place in a first rotational direction) and then the threaded element 857 of FIG. 50 is fitted over the head 856 of the central fastening element 855 and installed. In such implementation of FIG. 50, the inside of the threaded element 857 is not threaded. Rather, the inside of the threaded element 857 of FIG. 50 can be a non-threaded cylindrical bore to slip over the head 856. This implementation of FIG. 50 can aid in minimizing micro motion between the central fastening element 855 and the threaded element 857, which can aid in preventing the central fastening element 855 from backing out of its installed position. The threaded element 857 of FIG. 49 or of FIG. 50 can be included in any of the glenoid implants of the present disclosure for the same or similar purposes as described herein.

The threaded element 857 is configured to be received within the central aperture 840 of the first portion 830, which is also threaded. The threaded element 857 is configured to lock the central fastening element 855 in place. As best shown in FIG. 48, the central aperture 851 of the glenosphere 850 can be configured to receive the first trunnion 837. In this way, the glenosphere 850 is secured to the base of the glenoid implant 800.

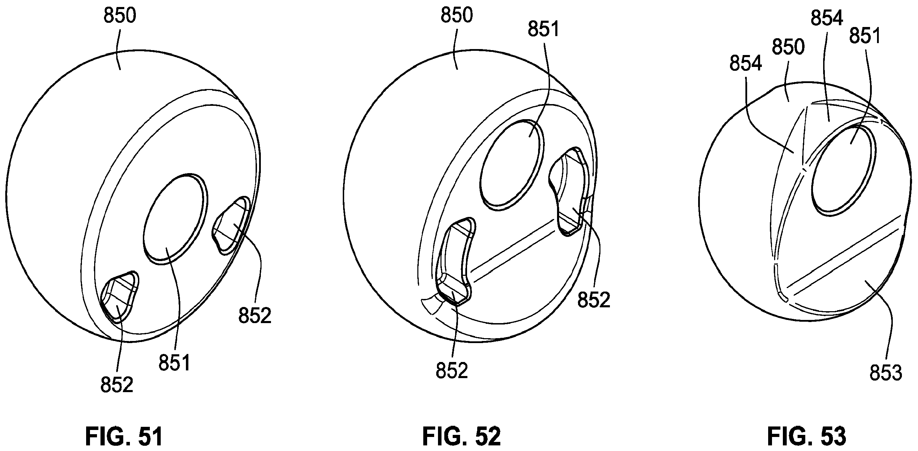

While the glenosphere 850 has been illustrated as discussed above, it should be understood that the geometry of the glenosphere 850 can also vary. FIGS. 51, 52 and 53 are rear isometric views of a glenosphere 850 in a first, second, and third configuration, respectively. FIG. 51 illustrates the glenosphere 850 with a central aperture 851 and receiving slots 852 for one or two augments (not shown). FIG. 52 illustrates the glenosphere 850 with a central aperture 851, receiving slots 852, and a tab or wedge feature 853. Due to the tab or wedge feature 853 location on the glenosphere 850 (FIG. 52), the position and size of the receiving slots 852 of the second configuration varies with respect to that of the receiving slots 852 of the first configuration (FIG. 51). FIG. 53 illustrates the glenosphere 850 with a central aperture 851, a first tab or wedge feature 853, and a second tab or wedge feature 854 that is opposite the first tab or wedge feature 853. These tab/wedge features can assist in securing the glenosphere 850 to the other elements of the glenoid implants of the present disclosure. They can also provide additional support to the scapula.

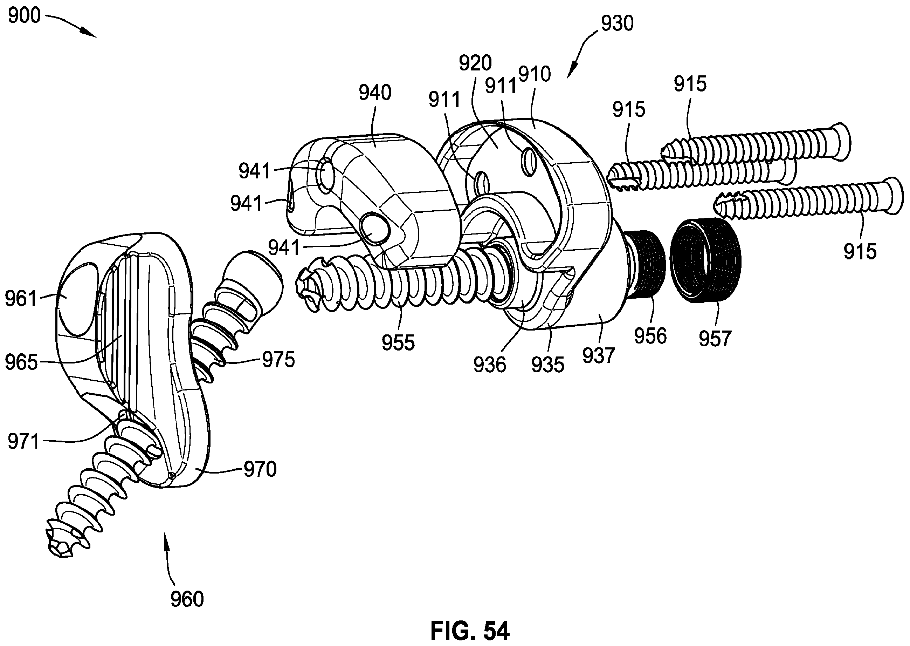

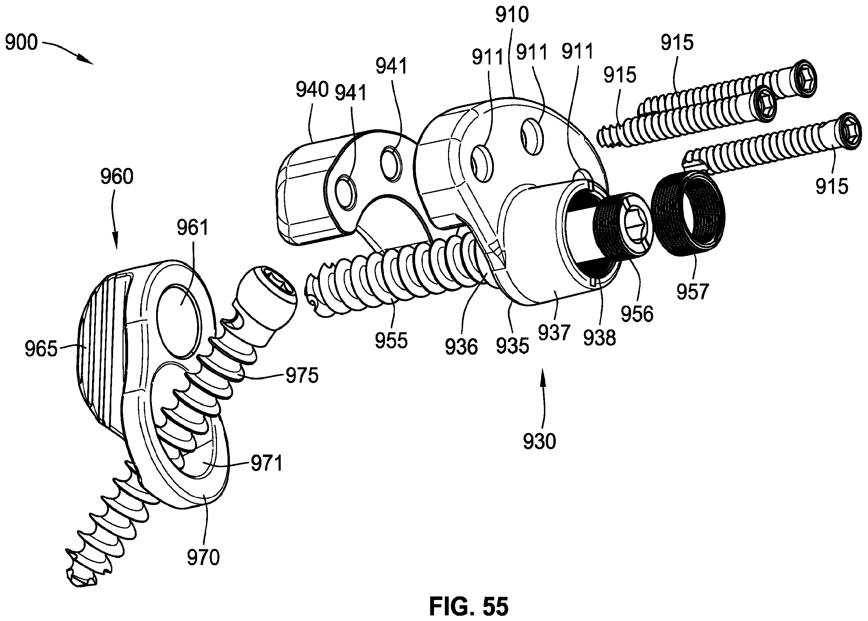

Referring generally to FIGS. 54-55, a glenoid implant 900 is illustrated. FIG. 54 is a rear isometric or perspective exploded view of the glenoid implant 900, whereas FIG. 55 is a front isometric or perspective exploded view of the glenoid implant 900. The glenoid implant 900 includes a base element 960, a coupling element 930, and an augment 940. The base element 960 has a central aperture 961 and an adjustable third leg 970, which includes a second aperture 971. The adjustable third leg 970 is adjustable relative to the coupling element 930 in that the adjustable third leg 970 can be rotated relative to the coupling element 930. The base element 960 includes a keel element 965 extending or protruding from a rear of the base element 960. The keel element 965 is configured to aid in securing the base element 960 to a first portion of a scapula of a patient/user. The second aperture 971 of the base element 960 is configured to receive a portion of a first fastening element 975 therethrough to aid in securing the base element 960 to the first portion of the scapula. In some implementations, the first portion of the scapula is a glenoid cavity. The coupling element 930 includes a first elongated portion 910 and a second portion 935. The first elongated portion 910 includes a receiving space 920 (FIG. 54), and apertures 911 therethrough. Furthermore, the second portion 935 includes a central aperture 938 that is aligned with the central aperture 961 of the base element 960 when the base element 960 is coupled to the coupling element 930.

The augment 940 is sized and configured to be at least partially received within the receiving space 920 of the first elongated portion 910 of the coupling element 930. The augment 940 includes apertures 941 therethrough. Each aperture 911 of the coupling element 930 is aligned with each aperture 941 of the augment 940 to receive a portion of a respective second fastening element 915 therethrough. The second fastening elements 915 can aid in securing the coupling element 930 and/or the augment 940 to a second portion of the scapula 50 of the patient/user.

The second portion 935 of the coupling element 930 can include a first trunnion 937 and a second trunnion 936 opposite the first trunnion 937. The first trunnion 937 is configured to receive or be received in a glenosphere (not shown). The second trunnion 936 can be configured to be received within the central aperture 961 (FIG. 55) of the base element 960. As a result, the coupling element 960 and the augment 940, once coupled, are configured to rotate relative to the base element 960 and/or relative to an axis of the base element 960 (e.g., a central axis of the central aperture 961). While three second fastening elements 915 are illustrated herein, it should be understood that not all the second fastening elements 915 need be included if, for example, the coupling element 960 and the augment 940 are rotated to one side. The glenoid implant 900 can be implemented in cases of severe bone loss. The base element 960 can be configured as an inlay component, meaning, the keel element 965 can be shaped to account for a cavity defect or missing bone.

The glenoid implant 900 can also include a central fastening element 955 configured to be at least partially positioned through the central aperture 961 of the base element 960 and the central aperture 938 of the coupling element 930. The central fastening element 955 is similar in variations with the central fastening element 855 of the glenoid implant 800.

A head 956 of the central fastening element 955 can be the same as or similar to the head 856 described above. Similarly, the glenoid implant 900 can include a threaded element 957 that is the same as, or similar to, the threaded element 857 described above.

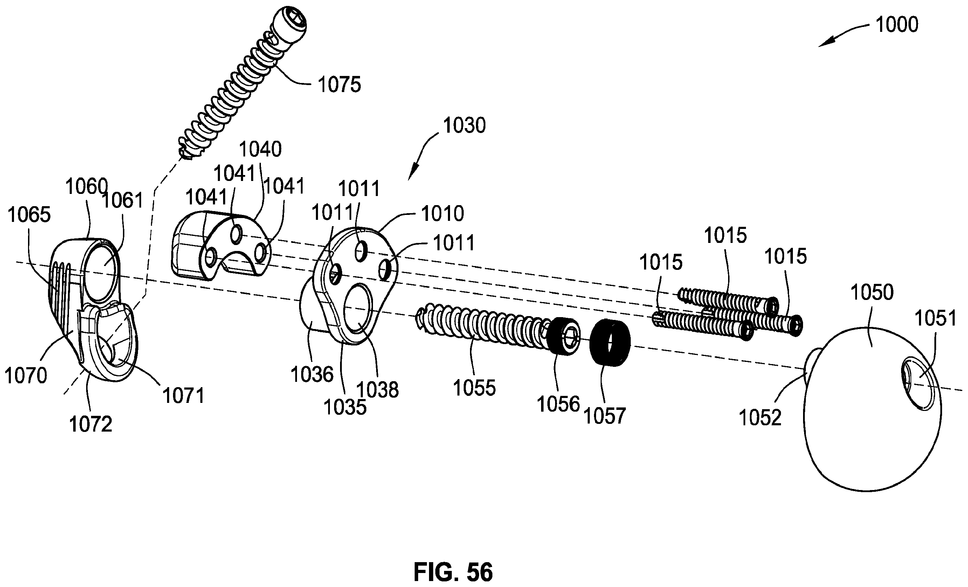

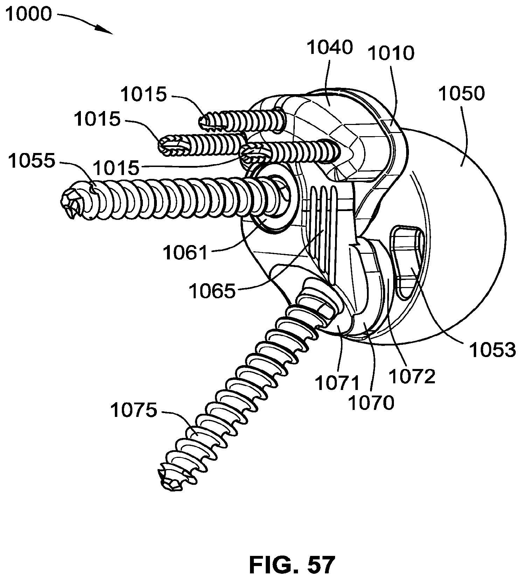

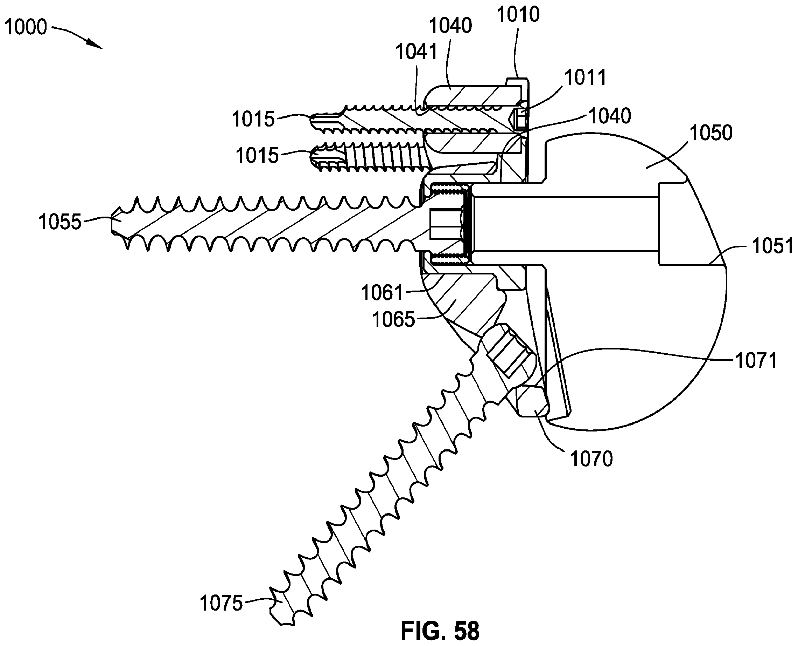

Referring generally to FIGS. 56-58, a glenoid implant 1000 is illustrated. FIG. 56 is a front isometric exploded view of the glenoid implant 1000. FIG. 57 is a right rear isometric view of the glenoid implant 1000. FIG. 58 is a cross-sectional side view of the glenoid implant 1000.

The glenoid implant 1000 can include a base element 1060, a coupling element 1030, an augment 1040, a central fastening element 1055 with a threaded head 1056 and a threaded element 1057, a first fastening element 1075, and second fastening elements 1015, which are similar to, the base element 960, the coupling element 930, the augment 940, the central fastening element 955 with the threaded head 956 and threaded element 957 of the glenoid implant 900, the first fastening element 975, and the second fastening elements 915. Specific to the glenoid implant 1000, the coupling element 1030 can have a shallower receiving area (not shown) than that of the coupling element 930. The coupling element 1030 includes a first elongated portion 1010 and a second portion 1035. The first elongated portion 1010 includes a receiving space that receives the augment 1040 therein as shown in FIG. 57. The first elongated portion 1010 also includes apertures 911 therethrough for permitting the second fastening elements 1015 to at least partially pass therethrough for securing the glenoid implant 1000 during installation. Furthermore, the second portion 1035 includes a central aperture 1038 that is aligned with the central aperture 1061 of the base element 1060 when the base element 1060 is coupled to the coupling element 1030.

The augment 1040 includes apertures 1041 therethrough. Each aperture 1011 of the coupling element 1030 is aligned with each aperture 1041 of the augment 1040 to receive a portion of a respective one of the second fastening elements 1015 therethrough. The second fastening elements 1015 can aid in securing the coupling element 1030 and/or the augment 1040 to a second portion of the scapula 50 of the patient/user.

Furthermore, the base element 1060 can include a tab or wedge feature 1072 extending from the aperture 1071 of the adjustable third leg 1070. Moreover, the coupling element 1030 can have a sole trunnion 1036 extending from the rear, and configured to be received within the central aperture 1061 of the base element 1060. Like the base element 960, the base element 1060 includes a keel element 1065 that extends or protrudes from a rear of the base element 1060.

The glenoid implant also includes a glenosphere 1050 that is connected to the coupling element 1030 via a trunnion 1052 of the glenosphere 1050 that extends from the glenosphere 1050. The trunnion 1052 can be configured to be received within the central aperture 1038 of the coupling element 1030. The glenosphere 1050 can include one or more receiving spaces 1053 (FIG. 57) configured to receive one or more corresponding augments (not shown, but could be the same as, or similar to, augments 804). The augments in the bottom of the glenosphere 1050 can be attached with a screw or the augments can be tapered and hammered into the glenosphere 1050. The glenosphere 1050 also includes a central aperture 1051 (FIG. 56) that permits the central fastening element 1055 and/or the threaded element 1057 to be passed therethrough (at least partially) during installation of the glenoid implant 1000 into bone of a patient.

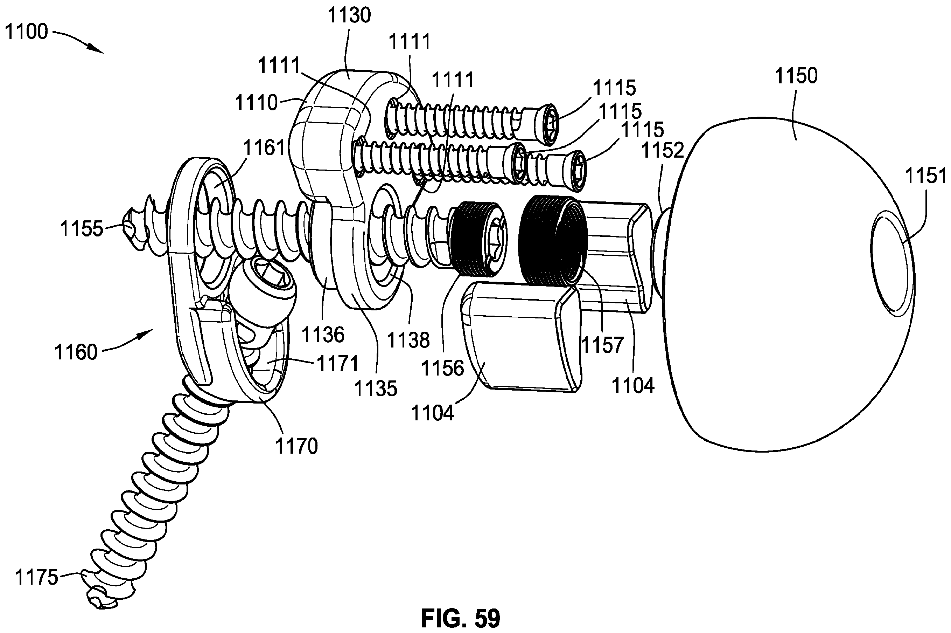

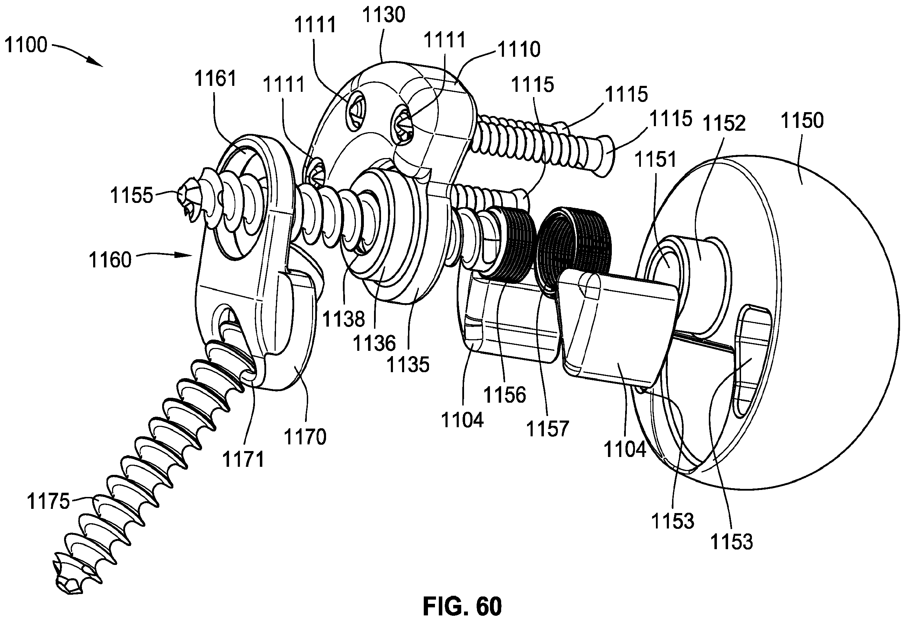

Referring generally to FIGS. 59-60, a glenoid implant 1100 is illustrated. FIGS. 59 and 60 are front and rear isometric exploded views of the glenoid implant 1100. The glenoid implant 1100 includes a base element 1160, a coupling element 1130, a central fastening element 1155, a first fastening element 1175, and second fastening elements 1115, which are the same as, or similar to, the base element 1060, the coupling element 1030, the central fastening element 1055 of the glenoid implant 1000, the first fastening element 1075, and the second fastening elements 1015. The base element 1160 has a central aperture 1161 and an adjustable third leg 1170, which includes a second aperture 1171. The adjustable third leg 1170 is adjustable relative to the coupling element 1130 in that the adjustable third leg 1170 can be rotated relative to the coupling element 1130. The second aperture 1171 of the base element 1160 is configured to receive a portion of a first fastening element 1175 therethrough to aid in securing the base element 1160 to the first portion of the scapula 50.

The coupling element 1130 includes a first elongated portion 1110 and a second portion 1135 (FIG. 60). The first elongated portion 1110 includes apertures 1111 therethrough. Furthermore, the second portion 1135 includes a central aperture 1138 that is aligned with the central aperture 1161 of the base element 1160 when the base element 1160 is coupled to the coupling element 1130. Specific to the glenoid implant 1100, the coupling element 1130 does not have a receiving area because there is no augment component to attach. The first elongated portion 1110 of the coupling element 1130 has an extended geometrical shape, and is not flat like its predecessors discussed above. Furthermore, a second trunnion 1136 is relatively shorter than the trunnion 1036 discussed above. This is because the base component 1160 has a slimmer profile than the base component 1060 discussed above.

The base element 1160 has a flat rear surface, as it is configured as an on-lay component. The glenoid implant also includes a glenosphere 1150 that is connected to the coupling element 1130 via a trunnion 1152 of the glenosphere 1150 that extends from the glenosphere 1150. The trunnion 1152 can be configured to be received within the central aperture 1138 of the coupling element 1130. The glenosphere 1150 can include one or more receiving spaces 1153 (FIG. 60) configured to receive one or more corresponding augments (not shown, but could be the same as, or similar to, augments 804). The augments in the bottom of the glenosphere 1150 can be attached with a screw or the augments can be tapered and hammered into the glenosphere 1150. The glenosphere 1150 also includes a central aperture 1151 (FIGS. 59-60) that permits the central fastening element 1155 and/or the threaded element 1157 to be passed therethrough (at least partially) during installation of the glenoid implant 1100 into bone of a patient.

A head 1156 of the central fastening element 1155 can be the same as or similar to the head 856 described above. Similarly, the glenoid implant 1100 can include a threaded element 1157 that is the same as, or similar to, the threaded element 857 described above.

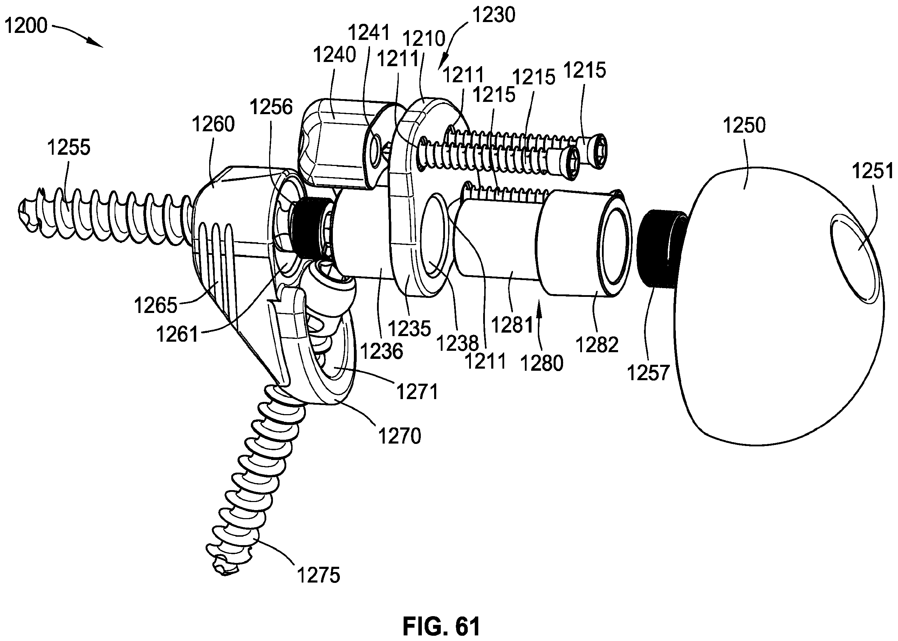

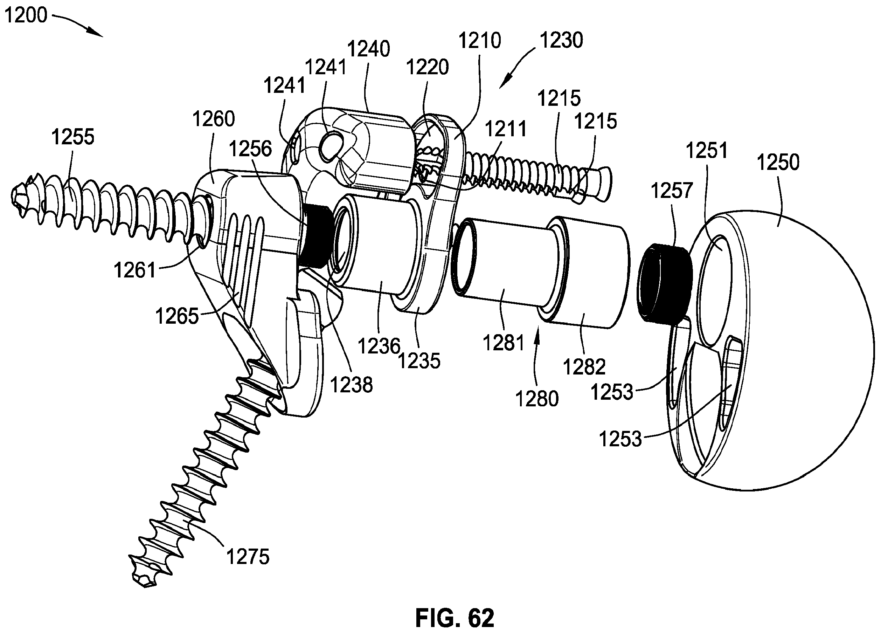

Referring generally to FIGS. 61-62, a glenoid implant 1200 is illustrated. FIG. 61 is a front isometric or perspective exploded view of the glenoid implant 1200, whereas FIG. 62 is a rear isometric of perspective exploded view of the glenoid implant 1200. The glenoid implant 1200 includes a base element 1260, a coupling element 1230, and an augment 1240, which are the same as, or similar to, the base element 960, the coupling element 930, and the augment 940 of the glenoid implant 900.

The base element 1260 has a central aperture 1261 and an adjustable third leg 1270, which includes a second aperture 1271. The adjustable third leg 1270 is adjustable relative to the coupling element 1230 in that the adjustable third leg 1270 can be rotated relative to the coupling element 1230. The base element 1260 includes a keel element 1265 extending or protruding from a rear of the base element 1260. The keel element 1265 is configured to aid in securing the base element 1260 to a first portion of a scapula of a patient/user. The second aperture 1271 of the base element 1260 is configured to receive a portion of a first fastening element 1275 therethrough to aid in securing the base element 1260 to the first portion of the scapula. In some implementations, the first portion of the scapula 50 is a glenoid cavity. The coupling element 1230 includes a first elongated portion 1210 and a second portion 1235. The first elongated portion 1210 includes a receiving space 1220 (FIG. 62), and apertures 1211 therethrough. Furthermore, the second portion 1235 includes a central aperture 1238 that is aligned with the central aperture 1261 of the base element 1260 when the base element 1260 is coupled to the coupling element 1230.

The augment 1240 is sized and configured to be at least partially received within the receiving space 1220 of the first elongated portion 1210 of the coupling element 1230. The augment 1240 includes apertures 1241 therethrough. Each aperture 1211 of the coupling element 1230 is aligned with each aperture 1241 of the augment 1240 to receive a portion of a respective second fastening element 1215 therethrough. The second fastening elements 1215 can aid in securing the coupling element 1230 and/or the augment 1240 to a second portion of the scapula 50 of the patient/user.

The second portion 1235 of the coupling element 1230 includes a trunnion 1236, but compared to the glenoid implant 900, the glenoid implant 1200, does not include a first trunnion opposite the trunnion 1236 extending from the coupling element 1230 towards the glenosphere 1250. The trunnion 1236 can be configured to be received within the central aperture 1261 (FIG. 61) of the base element 1260. As a result, the coupling element 1230 and the augment 1240, once coupled, are configured to rotate relative to the base element 1260 and/or relative to an axis of the base element 1260 (e.g., a central axis of the central aperture 1261). While three second fastening elements 1215 are illustrated herein, it should be understood that not all the second fastening elements 1215 need be included if, for example, the coupling element 1260 and the augment 1240 are rotated to one side. The glenoid implant 1200 can be implemented in cases of severe bone loss. The base element 1260 can be configured as an inlay component, meaning, the keel element 1265 can be shaped to account for a cavity defect or missing bone.

Instead of the coupling element 1230 including a trunnion like the first trunnion 937 of the glenoid implant 900, the glenoid implant 1200 includes a double trunnion element 1280, which include a first end 1281 and a second end 1282. A central fastening element 1255 can be received through an opening 1251 in the glenosphere 1250 and positioned generally through the double trunnion element 1280 such that at least a portion of a head 1256 of the central fastening element 1255 is within the double trunnion element 1280. A threaded element 1257 can also be received through the opening 1251 of the glenosphere 1250 and generally positioned in the second end 1282 of the double trunnion element 1280. The threaded element 1257 is configured to aid in locking the double trunnion element 1280 to the coupling element 1230 and aid in preventing the central fastening element 1255 from backing out (e.g., backing out of the double trunnion element 1280 and/or the glenosphere 1250). The threaded element 1257 screws onto the head 1256 of the central fastening element 1255 and or to threads inside the second end 1282 of the double trunnion element 1280.

The glenosphere 1250 can include receiving elements 1253. The receiving elements 1253 are configured to receive augments (not shown, but could be the same as, or similar to, augments 804). The augments in the bottom of the glenosphere 1250 can be attached with a screw. Alternatively, the augments can be tapered and hammered into the glenosphere 1250 (e.g., a press fit or a Morse tapered fit, or the like).

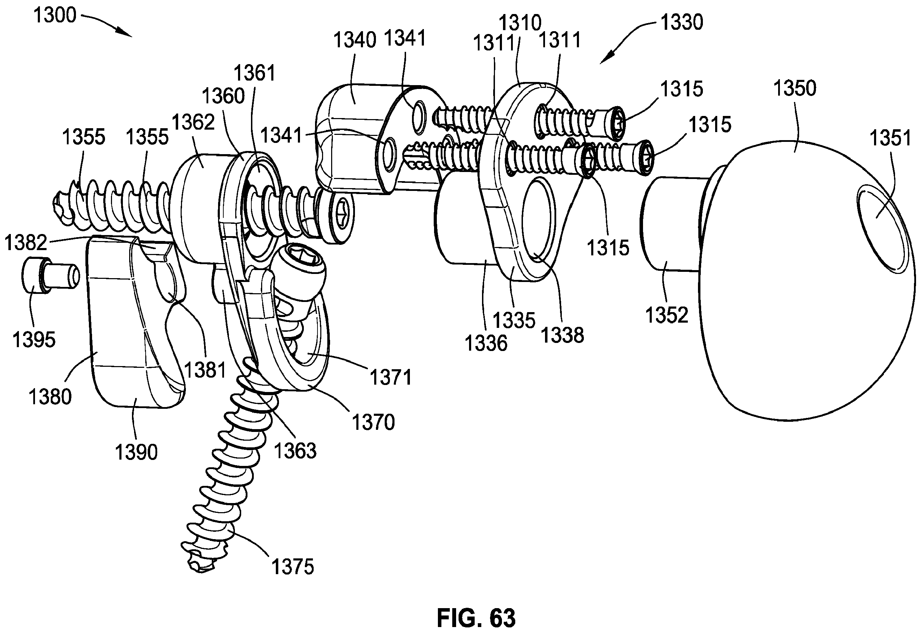

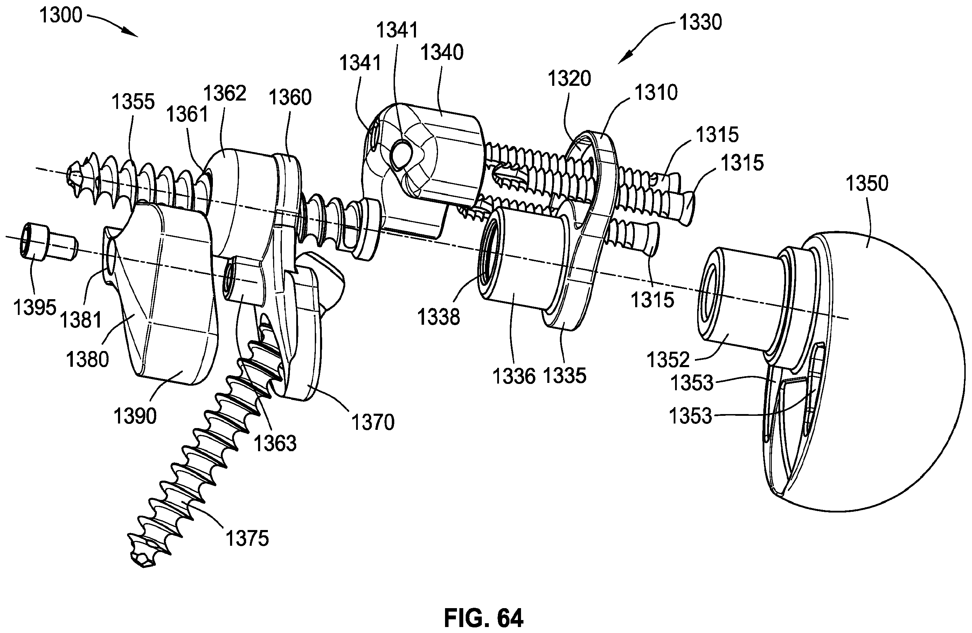

Referring generally to FIGS. 63-64, a glenoid implant 1300 is illustrated. FIG. 63 is a front isometric or perspective exploded view of the glenoid implant 1300, whereas FIG. 64 is a rear isometric of perspective exploded view of the glenoid implant 1300. The glenoid implant 1300 includes a base element 1360, a coupling element 1330, an augment 1340, and a glenosphere 1350, which are the same as, or similar to, the base element 1160, the coupling element 1230, the augment 1240, and the glenosphere 1250.

The base element 1360 has a central aperture 1361 and an adjustable third leg 1370, which includes a second aperture 1371. The adjustable third leg 1370 is adjustable relative to the coupling element 1330 in that the adjustable third leg 1370 can be rotated relative to the coupling element 1330. The base element 1360 includes a rear trunnion 1362 extending or protruding from a rear of the base element 1360. The rear trunnion 1362 is configured to abut a removable wedge augment 1380.

The base element 1360 includes a receiving element or an attachment portion 1363 that is positioned adjacent to the rear trunnion 1362. The removable wedge augment 1380 includes one or more abutting surfaces 1382 that are configured to abut one or more corresponding portions of the rear trunnion 1362 when the removable wedge augment 1380 is coupled to the base element 1360. As shown, the one or more abutting surfaces 1382 are generally curved in a manner to match and/or mate with the curved and/or cylindrical outer surface of the rear trunnion 1362. Alternatively, the one or more abutting surfaces 1382 can have any shape and/or curvature or be flat and/or angled relative to a central axis of a central aperture 1381.

The removable wedge augment 1380 includes the central aperture or slot or slit 1381 that is configured to receive a fastening element 1395 (e.g., a threaded fastening element, such as, for example, a screw or bolt, or the like) therethrough. That is, in some implementations, the central aperture 1381 is opened on one side or end so as to not be a through bore style aperture. The fastening element 1395, when positioned at least partially through the central aperture 1381, can engage (e.g., in a threaded manner) the attachment portion 1363 to hold the removable wedge augment 1380 onto and in engagement with the base element 1360.

The removable wedge augment 1380 also includes a wedge portion 1390 that can have any size and/or shape so as to modify the overall shape and size of the base element 1360 when the removable wedge augment 1380 is coupled thereto. As such, the removable wedge augment 1380 can be coupled to the base element 1360 to provide the base element 1360 with a unique geometric shape configured to be inserted into a cavity defect of a scapula. Various shapes and sizes of the removable wedge augment 1380 can be provided, for example, in a kit, to provide various combinations for the overall shape and size of the glenoid implant 1300.

The second aperture 1371 of the base element 1360 is configured to receive a portion of a first fastening element 1375 therethrough to aid in securing the base element 1360 to the first portion of the scapula. In some implementations, the first portion of the scapula 50 is a glenoid cavity. The coupling element 1330 includes a first elongated portion 1310 and a second portion 1335. The first elongated portion 1310 includes a receiving space 1320 (FIG. 64), and apertures 1311 therethrough. Furthermore, the second portion 1335 includes a central aperture 1338 that is aligned with the central aperture 1361 of the base element 1360 when the base element 1360 is coupled to the coupling element 1330.

The augment 1340 is sized and configured to be at least partially received within the receiving space 1320 of the first elongated portion 1310 of the coupling element 1330. The augment 1340 includes apertures 1341 therethrough. Each aperture 1311 of the coupling element 1330 is aligned with each aperture 1341 of the augment 1340 to receive a portion of a respective second fastening element 1315 therethrough. The second fastening elements 1315 can aid in securing the coupling element 1330 and/or the augment 1340 to a second portion of the scapula 50 of the patient/user.

The second portion 1335 of the coupling element 1330 includes a trunnion 1336, but compared to the glenoid implant 900, the glenoid implant 1300, does not include a first trunnion opposite the trunnion 1336 extending from the coupling element 1330 towards the glenosphere 1350. The trunnion 1336 can be configured to be received within the central aperture 1361 (FIG. 63) of the base element 1360. As a result, the coupling element 1330 and the augment 1340, once coupled, are configured to rotate relative to the base element 1360 and/or relative to an axis of the base element 1360 (e.g., a central axis of the central aperture 1261). While three second fastening elements 1315 are illustrated herein, it should be understood that not all the second fastening elements 1315 need be included if, for example, the coupling element 1360 and the augment 1340 are rotated to one side. The glenoid implant 1300 can be implemented in cases of severe bone loss. The base element 1360 can be configured as an inlay component, meaning, the removable wedge augment 1380 can be shaped to account for a cavity defect or missing bone.

Instead of the coupling element 1330 including a trunnion like the first trunnion 937 of the glenoid implant 900, the glenosphere 1350 includes a trunnion element 1352 that engages with the central aperture 1338 of the second portion 1335 of the coupling element 1330.

A central fastening element 1355 can be received through an opening 1351 in the glenosphere 1350 and positioned generally through the trunnion element 1352 such that at least a portion of a head of the central fastening element 1355 is within the trunnion 1336 and/or the central aperture 1361 of the base element 1360.

The glenosphere 1350 can include receiving elements 1353. The receiving elements 1353 are configured to receive augments (not shown, but could be the same as, or similar to, augments 804). The augments in the bottom of the glenosphere 1350 can be attached with a screw. Alternatively, the augments can be tapered and hammered into the glenosphere 1350 (e.g., a press fit or a Morse tapered fit, or the like).

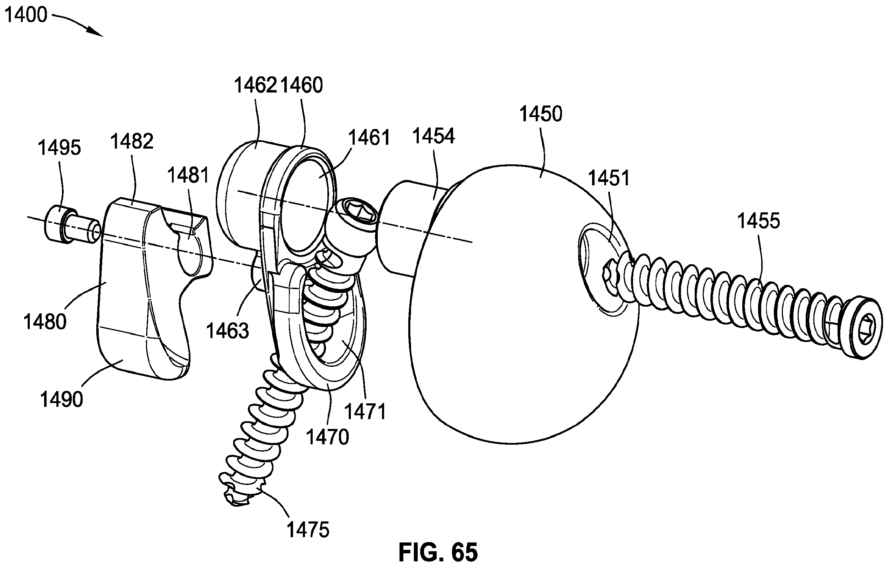

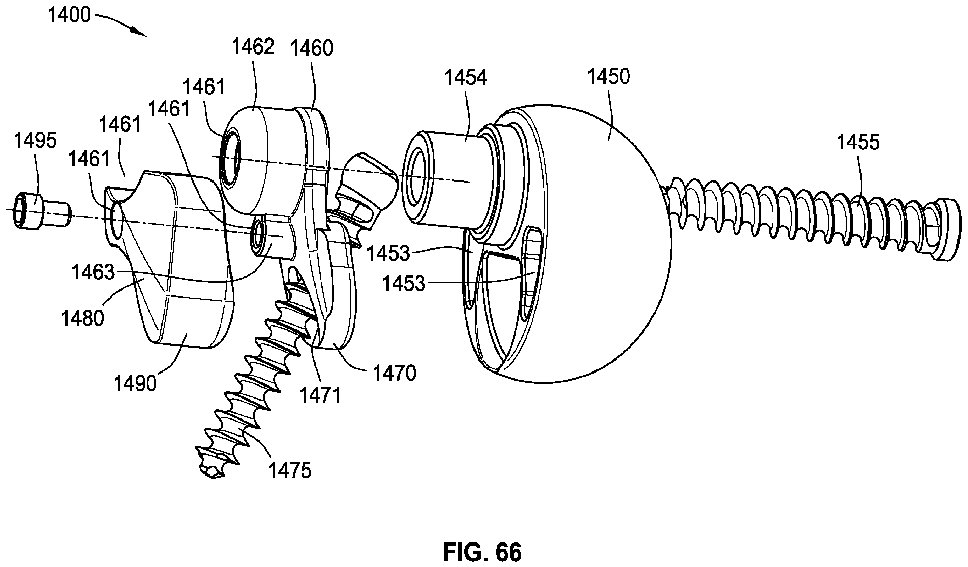

Referring generally to FIGS. 65-66, a glenoid implant 1400 is illustrated. FIG. 65 is a front isometric or perspective exploded view of the glenoid implant 1400, whereas FIG. 66 is a rear isometric of perspective exploded view of the glenoid implant 1400. The glenoid implant 1400 includes a base element 1460, a removable wedge augment 1480, and a glenosphere 1450, which are the same as, or similar to, the base element 1360, the removable wedge augment 1380, and the glenosphere 1350 of the glenoid implant 1300.

The base element 1460 has a central aperture 1461 and an adjustable leg 1470, which includes a second aperture 1471. The adjustable leg 1470 is adjustable relative to the glenosphere 1450 in that the adjustable leg 1470 can be rotated relative to the trunnion 1454. The base element 1460 includes a rear trunnion 1462 extending or protruding from a rear of the base element 1460. The rear trunnion 1462 is configured to abut the removable wedge augment 1480.

The glenoid implant 1400 differs from the glenoid implant 1300 in that the glenoid implant 1400 does not include a coupling element (e.g., coupling element 1330), nor does the glenoid implant 1400 include an augment (e.g., augment 1340). These elements are eliminated from the glenoid implant 1400 at least in part because the glenosphere 1450 is directly coupled with the base element 1460. Specifically, a trunnion 1454 of the glenosphere 1450 is coupled to the central aperture 1461 of the rear trunnion 1462 of the base element 1460.

Like the removable wedge augment 1380, the removable wedge augment 1480 includes a central aperture or slot or slit 1481, one or more abutting surfaces 1482, a wedge portion 1490, and a fastening element 1495 that are the same as, or similar to, the central aperture or slot or slit 1381, the one or more abutting surfaces 1382, the wedge portion 1390, and the fastening element 1395 of the removable wedge augment 1380. Further, the base element 1460 includes an attachment portion 1463 that can receive a portion of the fastening element 1495 to aid in holding the removable wedge augment 1480 in engagement with the base element 1460.

The glenoid implant 1400 also includes a first fastening element 1475 and a central fastening element 1455 that are the same as, or similar to, the first fastening element 1375 and the central fastening element 1355 of the glenoid implant 1300. The central fastening element 1455 can be received through an opening 1451 in the glenosphere 1450 and positioned generally through the rear trunnion 1462 such that at least a portion of a head of the central fastening element 1455 is within the trunnion 1454 and/or the central aperture 1461 of the base element 1460.

The glenosphere 1450 can include receiving elements 1453. The receiving elements 1453 are configured to receive augments (not shown, but could be the same as, or similar to, augments 804). The augments in the bottom of the glenosphere 1450 can be attached with a screw. Alternatively, the augments can be tapered and hammered into the glenosphere 1450 (e.g., a press fit or a Morse tapered fit, or the like).

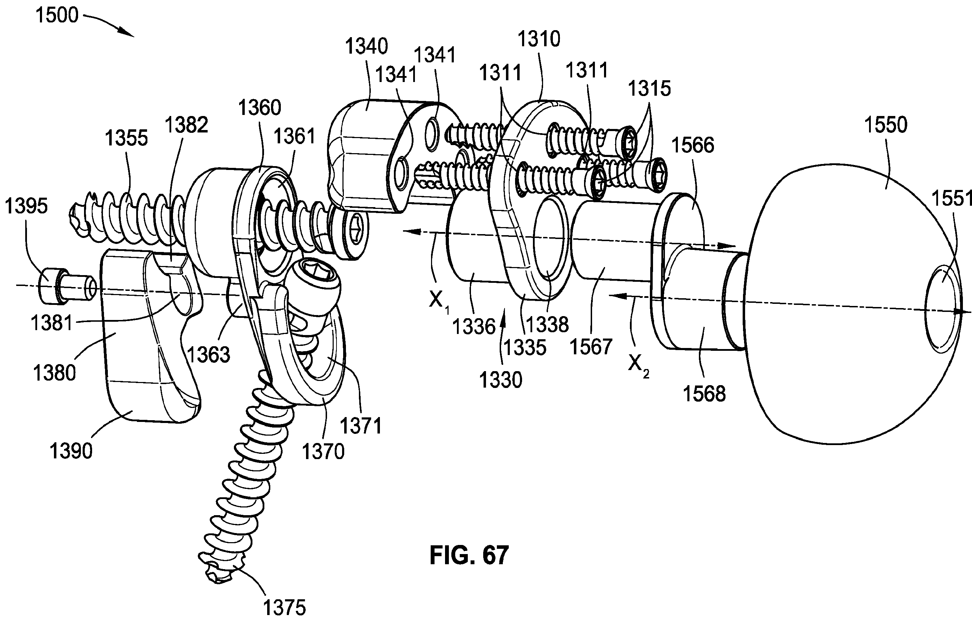

Referring generally to FIGS. 67-68, a glenoid implant 1500 is illustrated, which is the same as, or similar to, the glenoid implant 1300 except for the trunnion element 1352 of the glenosphere 1350 is replaced with central aperture 1551 of a glenosphere 1550, and an offset element 1566 is included. As such, like references are used for like elements and the disclosure for such like elements is not repeated for the glenoid implant 1500.

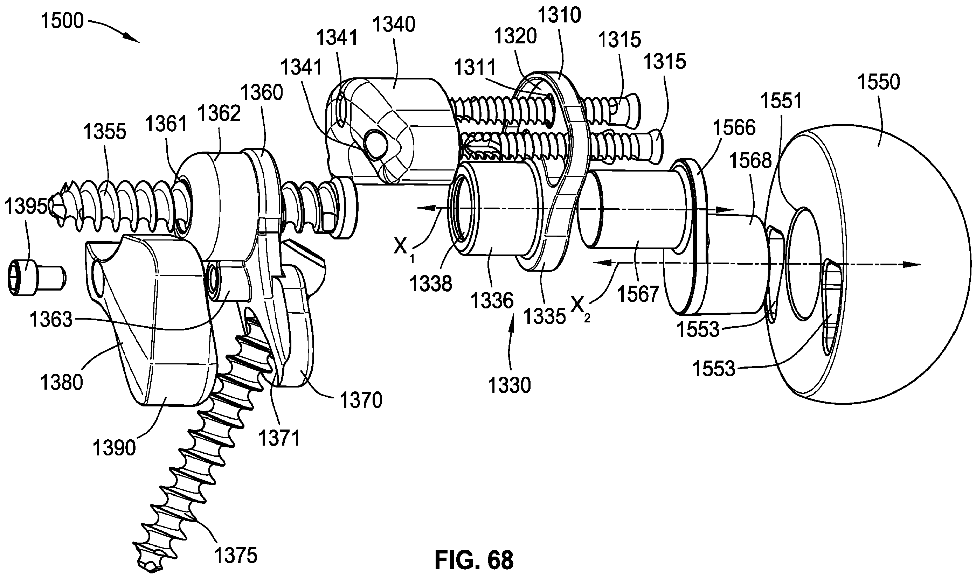

FIG. 67 is a front isometric or perspective exploded view of the glenoid implant 1500, whereas FIG. 68 is a rear isometric of perspective exploded view of the glenoid implant 1500. The glenoid implant 1500 includes the base element 1360, the coupling element 1330, the augment 1340, and the removable wedge augment 1380, the glenosphere 1550, and the offset element 1566, where like references numbers from the glenoid implant 1300 are used for like components.

The glenosphere 1550 does not include a trunnion because the glenosphere 1550 is connected to the coupling element 1330 using the offset element 1566. The offset element 1566 includes a base with a first trunnion 1567 protruding from a first surface, and a second trunnion 1568 protruding from a second surface opposite the first surface. The first trunnion 1567 and the second trunnion 1568 have two distinct central axes that are offset from one another. The first trunnion 1567 is aligned with the central aperture 1338 of the coupling element 1330 and the central aperture 1361 of the base element 1360. The second trunnion 1568 is aligned with a central aperture 1551 of the glenosphere 1550. In this way, the glenosphere 1550 is coupled to the coupling element 1330 such that a central axis x₁ of the central aperture 1338 of the coupling element 1330 is not aligned with a central axis x₂ of the aperture 1551 within the glenosphere 1550.

The glenosphere 1550 can include receiving elements 1553. The receiving elements 1553 are configured to receive augments (not shown, but could be the same as, or similar to, augments 804). The augments in the bottom of the glenosphere 1550 can be attached with a screw. Alternatively, the augments can be tapered and hammered into the glenosphere 1550 (e.g., a press fit or a Morse tapered fit, or the like).

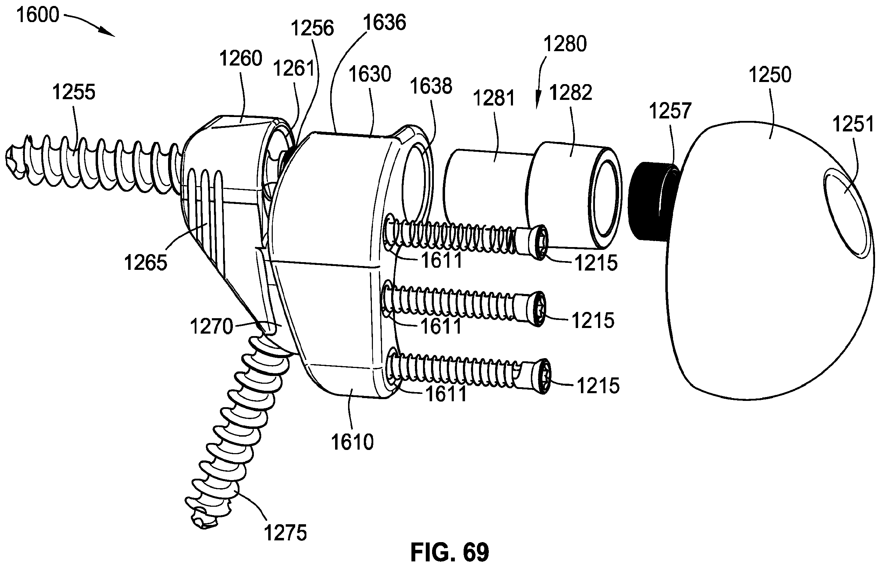

Referring generally to FIGS. 69-70, a glenoid implant 1600 is illustrated, which is the same as, or similar to, the glenoid implant 1200 except that the coupling element 1630 of the glenoid implant 1600 is different from the coupling element 1230 of the glenoid implant 1200. As such, like references are used for like elements and the disclosure for such like elements is not repeated for the glenoid implant 1600.

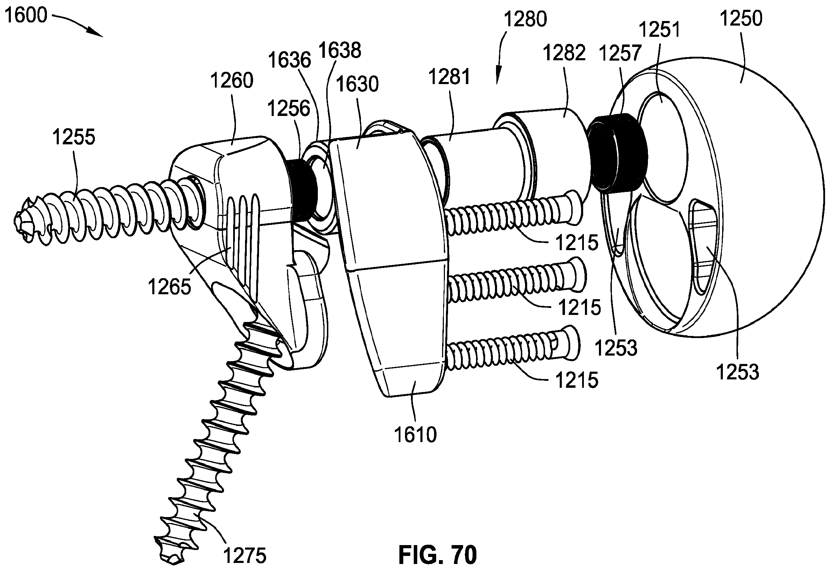

FIG. 69 is a front isometric or perspective exploded view of the glenoid implant 1600, whereas FIG. 70 is a rear isometric of perspective exploded view of the glenoid implant 1600. The glenoid implant 1600 includes the base element 1260, double trunnion element 1280, the glenosphere 1250, and the coupling element 1630, where like references numbers from the glenoid implant 1200 are used for like components.

The coupling element 1630 is generally position or biased to one side of the base element 1260. The coupling element 1630 includes a first elongated portion 1610 and a central aperture 1638. The elongated portion 1610 includes apertures 1611 therethrough, each configured to receive a portion of a second fastening element 1315. The coupling element 1630 also includes a trunnion 1636 configured to be inserted into the central aperture 1361 of the base element 1360.

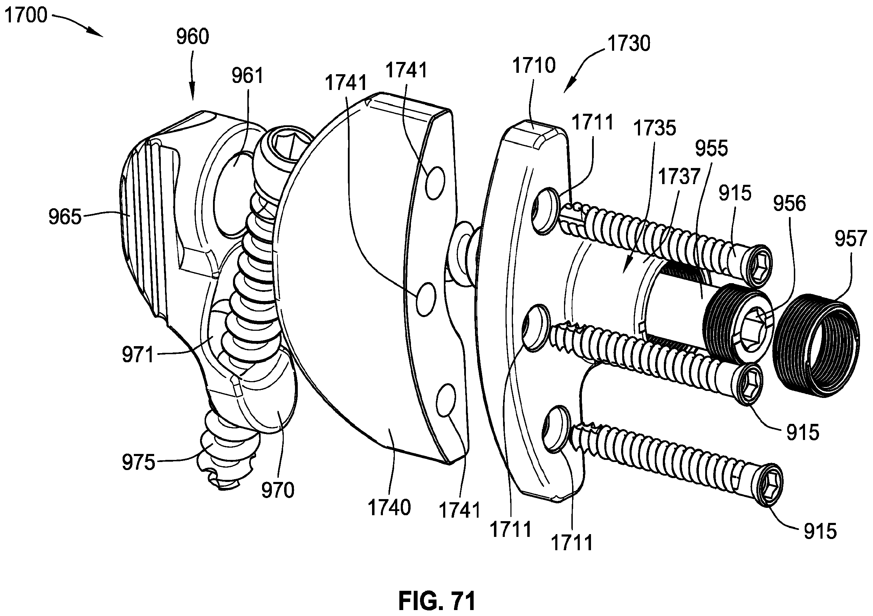

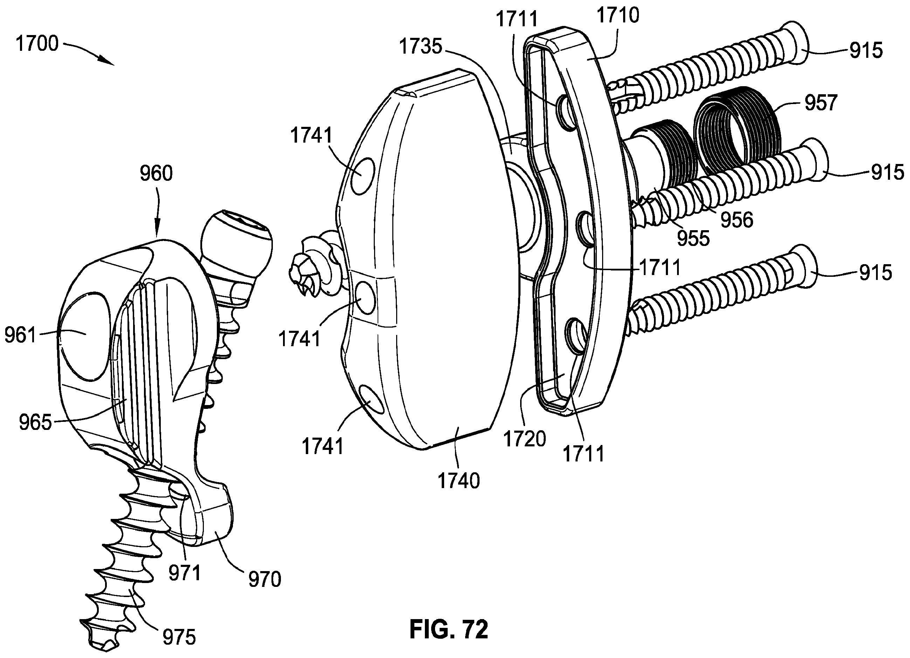

Referring generally to FIGS. 71-72, a glenoid implant 1700 is illustrated, which is the same as, or similar to, the glenoid implant 900 except that the coupling element 1730 of the glenoid implant 1700 is different from the coupling element 930 of the glenoid implant 900. As such, like references are used for like elements and the disclosure for such like elements is not repeated for the glenoid implant 1700.

FIG. 70 is a rear isometric or perspective exploded view of the glenoid implant 1700, whereas FIG. 71 is a front isometric of perspective exploded view of the glenoid implant 1700. The glenoid implant 1700 includes the base element 960, the first fastening element 975, the central fastening element 955, the threaded element 957, a coupling element 1630, and an augment 1740, where like reference numbers from the glenoid implant 900 are used for like components.

The coupling element 1730 and the augment 1740 are both generally positioned or biased to one side of the base element 960. The coupling element 1730 includes a first elongated portion 1710 and a second portion 1735. The elongated portion 1710 includes apertures 1711 therethrough, each configured to receive a portion of a second fastening element 915. The second portion 1735 of the coupling element 1730 includes and/or is a trunnion 1737 configured to be inserted into an aperture or cavity of a glenosphere (not shown). The elongated portion 1710 of the coupling element 1730 includes a receiving space 1720 configured to receive the augment 1740. The elongated portion 1710 has a relatively slimmer profile as compared to the elongated portion 1610 of the glenoid implant 1600. The augment 1740 includes apertures 1741 therethrough. Each aperture 1711 of the coupling element 1730 is aligned with each aperture 1741 of the augment 1740 to receive a portion of a respective second fastening element 915 therethrough.

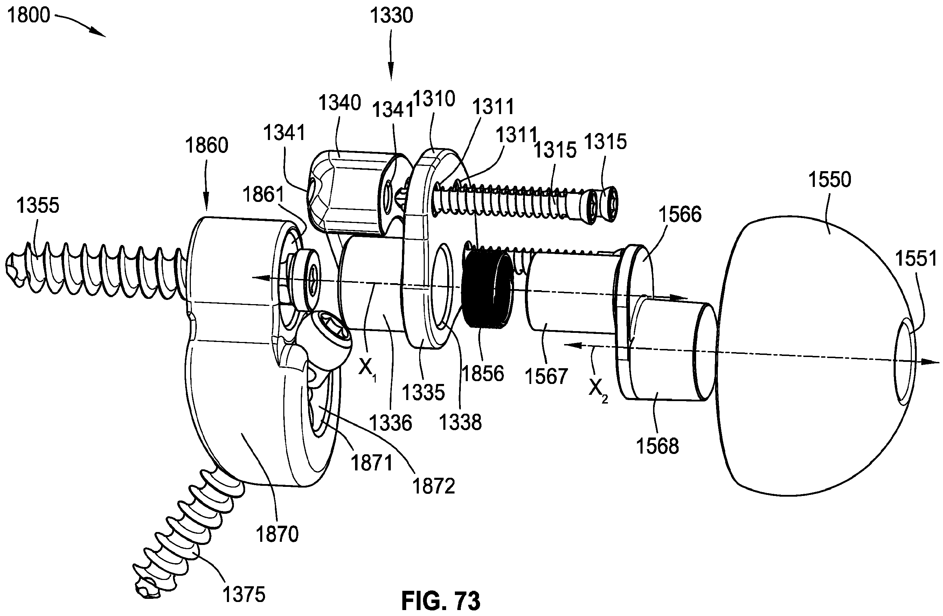

Referring generally to FIGS. 73-74, a glenoid implant 1800 is illustrated which is the same as, or similar to, the glenoid implant 1500 except for the base element 1360 is replaced with a base element 1860 and the removable wedge augment 1380 is not included in the glenoid implant 1800 and a threaded element 1856 is included. The threaded element 1856 is configured to engage threadingly with a corresponding threaded bore in the base element 1860. The threaded element 1856 aids in preventing the central fastening element 1355 from backing out (e.g., backing out into the first trunnion 1567 of the offset element 1566). As such, like references are used for like elements and the disclosure for such like elements is not repeated for the glenoid implant 1800. As shown, the threaded element 1856 is a cylindrical ring having an outer surface and an inner surface. The outer surface is threaded and the inner surface is threaded. In some implementations, the threaded outer surface is threaded in a first direction and the threaded inner surface is threaded in a second opposing direction. As such, the threaded element 1856 is able to be threaded onto a head of the central fastener element 1355 and into a threaded bore portion of the trunnion 1336. In some implementations, the threaded outer surface is threaded in a first direction and the threaded inner surface is threaded in first direction as well.

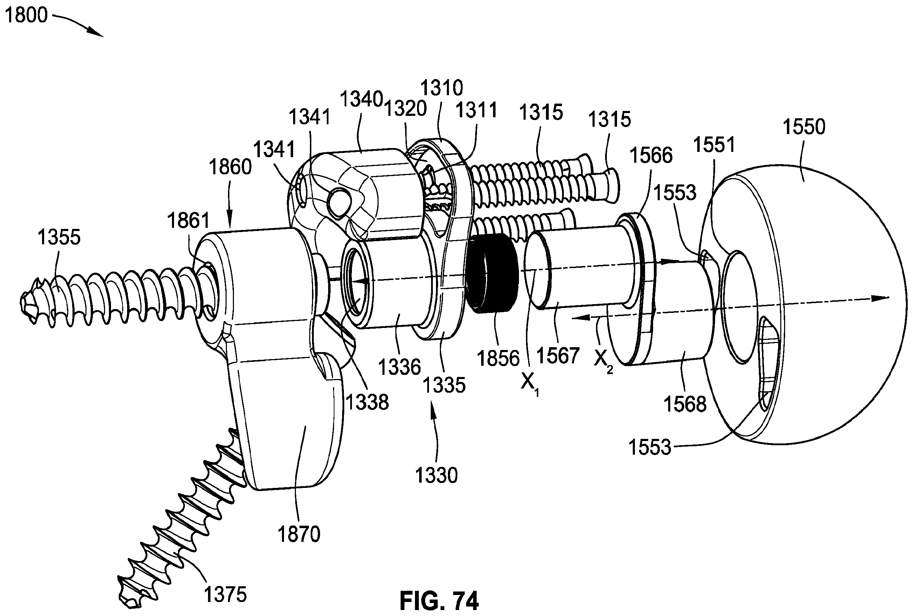

FIG. 73 is a front isometric or perspective exploded view of the glenoid implant 1800, whereas FIG. 74 is a rear isometric of perspective exploded view of the glenoid implant 1800. The glenoid implant 1800 includes the coupling element 1330, the augment 1340, the glenosphere 1550, the offset element 1566, and the base element 1860, where like references numbers from the glenoid implants 1300 and 1500 are used for like components.

The base element 1860 has a uni-body wedge shape. As best shown in FIG. 74, a left or first rear side 1870 of the base element 1860 is longer than an opposing right or second rear side 1872 of the base element 1860, to create a unique profile for a certain cavity defects. Put another way, a first portion of the base element 1860 surrounds a central aperture 1861 and a second portion of the base element 1860 surrounds a second aperture 1871. The second portion of the base element 1860 can be divided into two opposing sides, namely, the first or left side 1870 and the second or right side 1872 referenced above. As shown, the thickness or width of the first or left side 1870 is greater than the thickness or width of the second or right side 1872, which provides a shape of the base element with a generally wedge shape (e.g., from left to right, where the wedge thickness goes from relatively thick to relatively thinner).

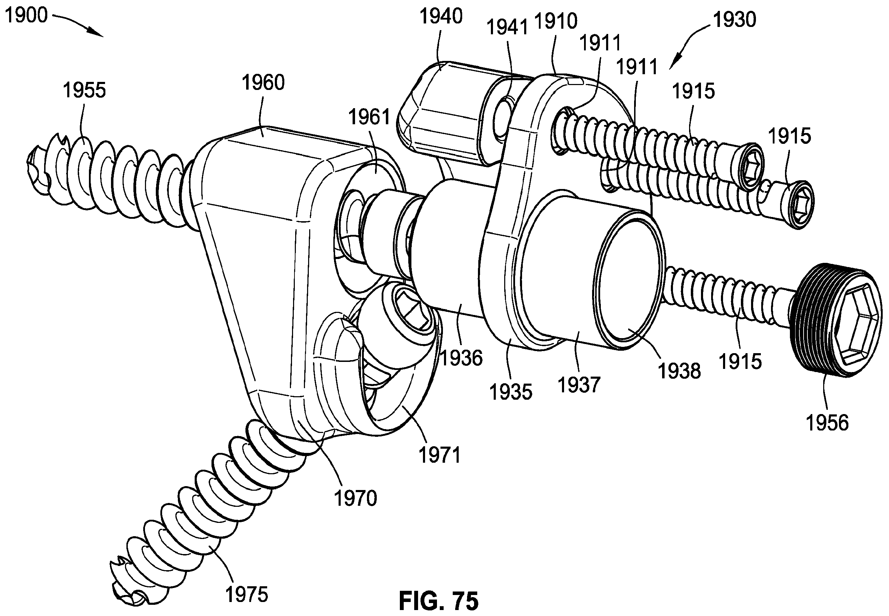

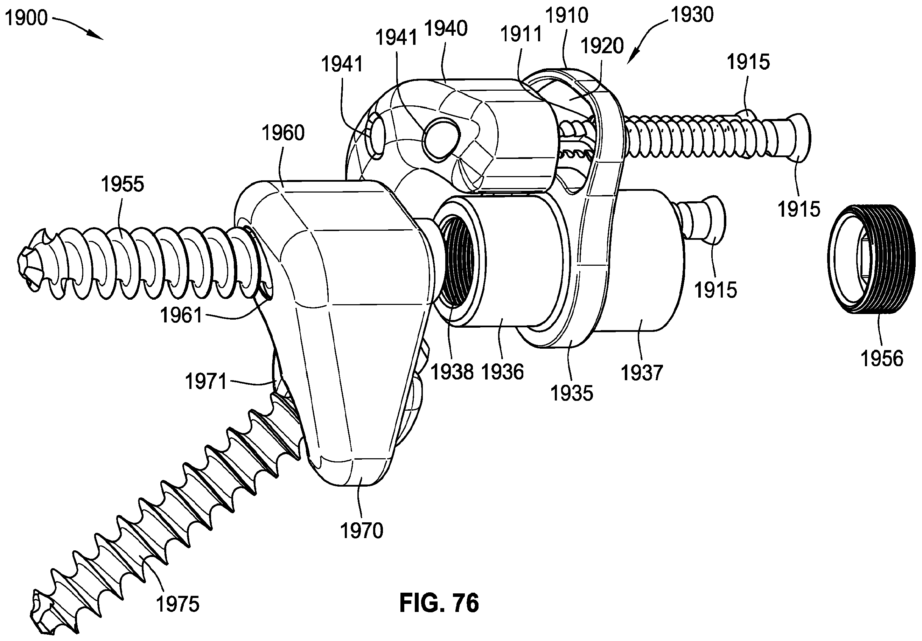

Referring generally to FIGS. 75-76, a glenoid implant 1900 is illustrated. FIG. 75 is a front isometric or perspective exploded view of the glenoid implant 1900, whereas FIG. 76 is a rear isometric or perspective exploded view of the glenoid implant 1900. The glenoid implant 1900 includes a base element 1960, a coupling element 1930, and an augment 1940. The base element 1960 has a central aperture 1961 and an adjustable third leg 1970, which includes a second aperture 1971. The adjustable third leg 1970 is adjustable relative to the coupling element 1930 in that the adjustable third leg 1970 can be rotated relative to the coupling element 1930. The base element 1960 is similar to the base element 960, except that the overall shape of the base element 1960 is a wedge shape as opposed to including a keel element, like the keel element 965 (FIG. 55). The second aperture 1971 of the base element 1960 is configured to receive a portion of a first fastening element 1975 therethrough to aid in securing the base element 1960 to the first portion of the scapula. In some implementations, the first portion of the scapula is a glenoid cavity. The coupling element 1930 includes a first elongated portion 1910 and a second portion 1935. The first elongated portion 1910 includes a receiving space 1920 (FIG. 76), and apertures 1911 therethrough. Furthermore, the second portion 1935 includes a central aperture 1938 that is aligned with the central aperture 1961 of the base element 1960 when the base element 1960 is coupled to the coupling element 1930.

The augment 1940 is sized and configured to be at least partially received within the receiving space 1920 of the first elongated portion 1910 of the coupling element 1930. The augment 1940 includes apertures 1941 therethrough. Each aperture 1911 of the coupling element 1930 is aligned with each aperture 1941 of the augment 1940 to receive a portion of a respective second fastening element 1915 therethrough. The second fastening elements 1915 can aid in securing the coupling element 1930 and/or the augment 1940 to a second portion of the scapula 50 of the patient/user.

The second portion 1935 of the coupling element 1930 can include a first trunnion 1937 and a second trunnion 1936 opposite the first trunnion 1937. The first trunnion 1937 is configured to receive or be received in a glenosphere (not shown). The second trunnion 1936 can be configured to be received within the central aperture 1961 (FIG. 75) of the base element 1960. As a result, the coupling element 1960 and the augment 1940, once coupled, are configured to rotate relative to the base element 1960 and/or relative to an axis of the base element 1960 (e.g., a central axis of the central aperture 1961). While three second fastening elements 1915 are illustrated herein, it should be understood that not all the second fastening elements 1915 need be included if, for example, the coupling element 1960 and the augment 1940 are rotated to one side. The glenoid implant 1900 can be implemented in cases of severe bone loss.

The glenoid implant 1900 can also include a central fastening element 1955 configured to be at least partially positioned through the central aperture 1961 of the base element 1960 and the central aperture 1938 of the coupling element 1930. The central fastening element 1955 is similar in variations with the central fastening element 1855 of the glenoid implant 800.

A head of the central fastening element 1955 can be the same as or similar to the head 856 described above. Similarly, the glenoid implant 1900 can include a threaded element 1956 that is the same as, or similar to, the threaded element 857 (or other threaded elements) described above.

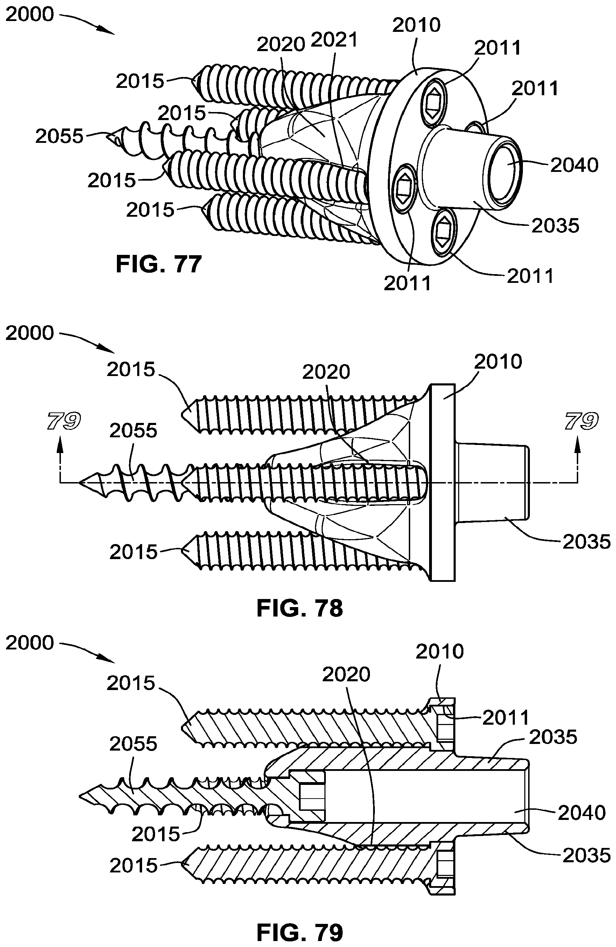

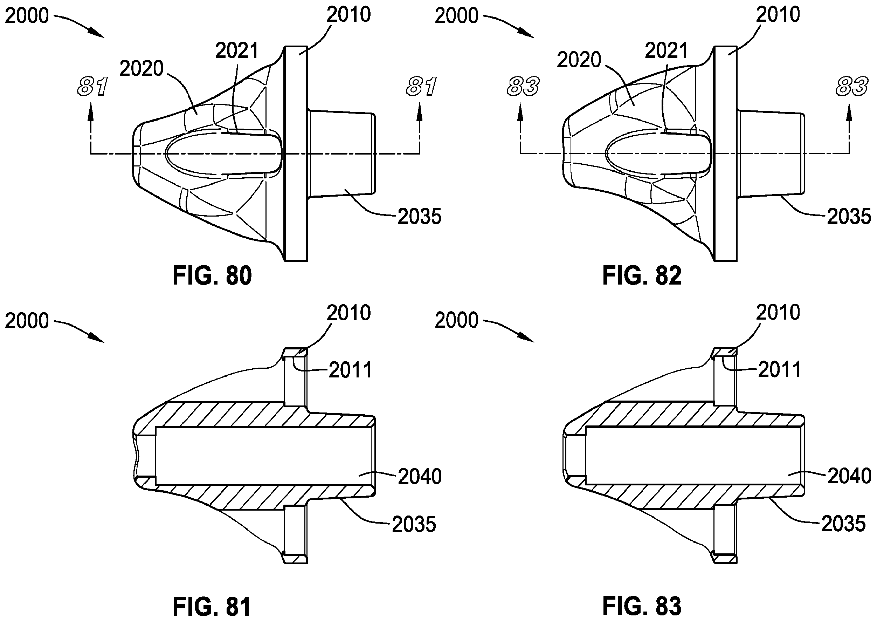

Referring generally to FIGS. 77-83, a glenoid implant 2000 is illustrated. FIG. 77 is a front isometric view of the glenoid implant 2000. FIG. 78 is a top view of the glenoid implant 2000 with cross-section 79-79. FIG. 79 is a side view of the glenoid implant 2000 at the cross-section 79-79. FIG. 80 is a first top view of the glenoid implant 2000 with a cross-section 81-81. FIG. 81 is a first side view of the glenoid implant 2000 at the cross-section 81-81. FIG. 82 is a second top view of the glenoid implant 2000 with a cross-section 83-83. FIG. 83 is the second side view of the glenoid implant 2000 at the cross-section 83-83.

The glenoid implant 2000 can include a baseplate 2010, a trunnion 2035 extending therefrom (e.g., for engaging a glenosphere), and a keel element 2020. The glenoid implant 2000 can include a central aperture 2040 therethrough configured to receive a central fastening element 2055 therethrough. The glenoid implant 2000 can also include apertures 2011 therethrough. Each of the apertures 2011 can be positioned around the central aperture 2040. The keel element 2020 can extend from a rear of the baseplate 2010. The central aperture 2040 can extend through the keel element 2020 such that the keel element 2020 and the baseplate 2010 are aligned. The keel element 2020 can also include grooves 2021. Each aperture 2011 of the baseplate 2010 can be aligned with each groove 2021 of the keel element 2020 to receive a portion of a second fastening element 2015 therethrough. The keel element 2020 is shaped to account for a cavity defect within the scapula of a patient.

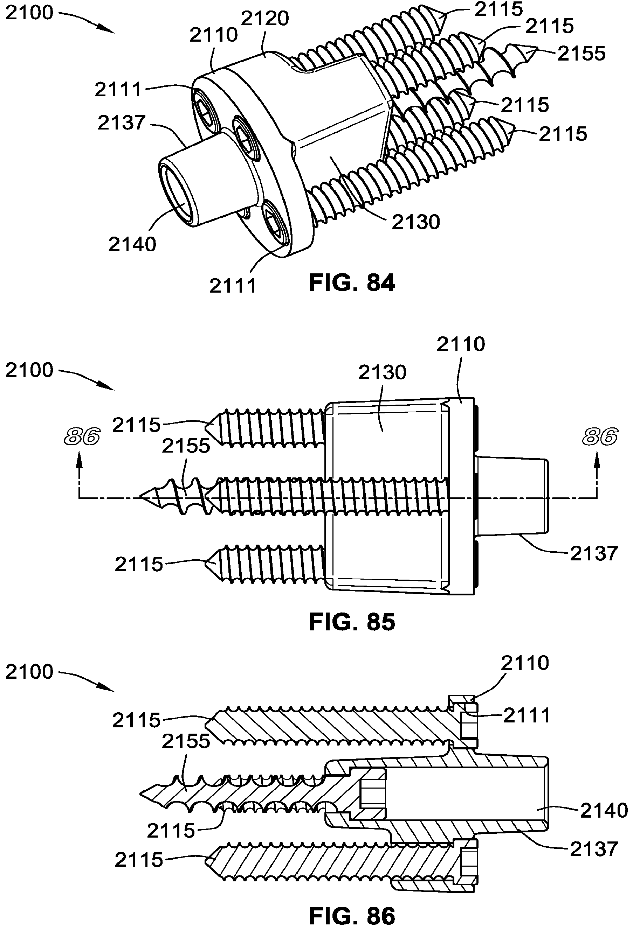

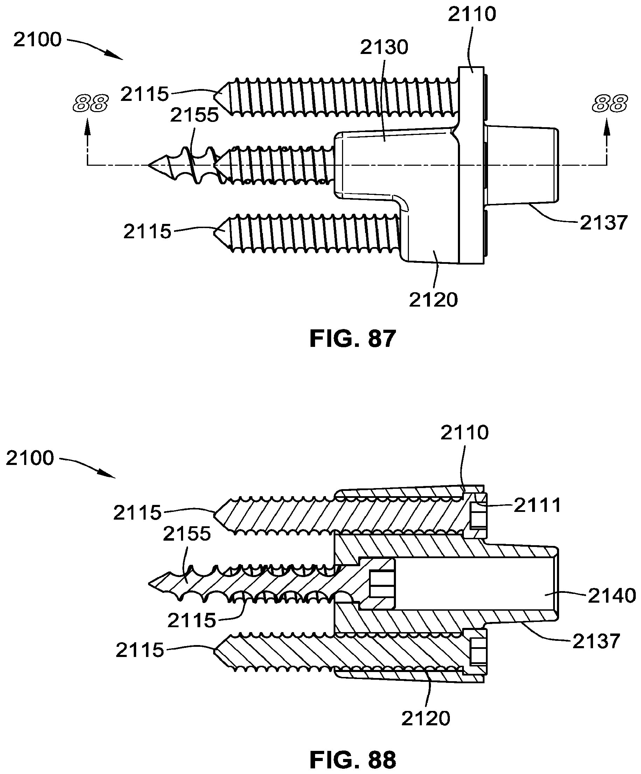

Referring generally to FIGS. 84-88, a glenoid implant 2100 is illustrated. FIG. 84 is a top isometric view of the glenoid implant 2100. FIG. 85 is a side view of the glenoid implant 2100 with a cross-section 86-86. FIG. 86 is the side view of the glenoid implant 2100 at the cross-section 86-86. FIG. 87 is a top view of the glenoid implant 2100 with a cross-section 88-88. FIG. 88 is the side view of the glenoid implant 2100 at the cross-section 88-88.

The glenoid implant 2100 includes a baseplate 2110, a trunnion 2137 extending therefrom (e.g., for engaging a glenosphere), a first keel portion 2120, and a second keel portion 2130. The glenoid implant 2100 can include a central aperture 2140 therethrough configured to receive a central fastening element 2155 therethrough.

The first keel portion 2120 and the second keel portion 2130 together form a stepped keel element. The first keel portion 2120 is generally adjacent to one side of the base plate 2110, whereas the second keel portion is generally adjacent to the center of the base plate 2110. In this way, at least one second fastening element 2115 will not pass through the keel element as best shown in FIGS. 84 and 85.

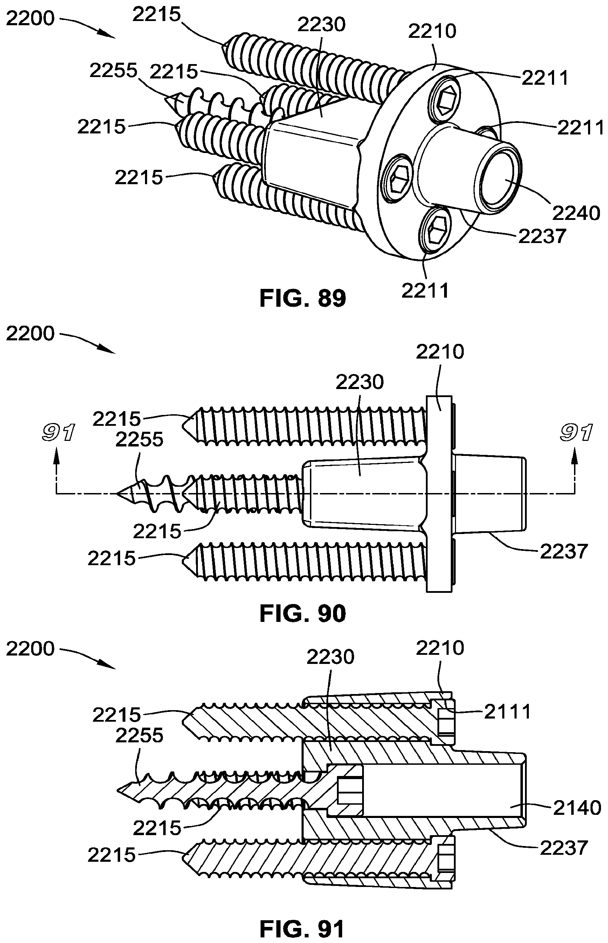

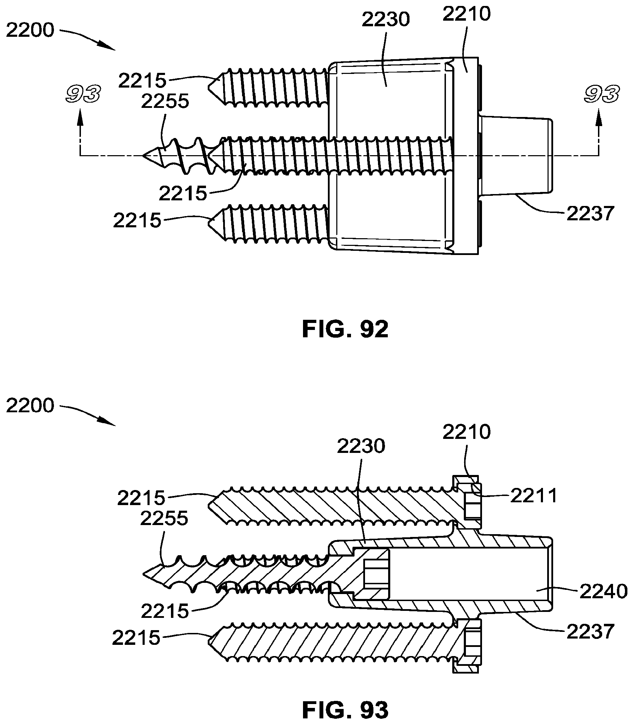

Referring generally to FIGS. 89-93, a glenoid implant 2200 is illustrated. FIG. 89 is a perspective view of the glenoid implant 2200. FIG. 90 is a first side view of the glenoid implant 2200. FIG. 91 is a side cross-sectional view of the glenoid implant 2200 at the cross-section 91-91 in FIG. 90. FIG. 92 is a second side view of the glenoid implant 2200. FIG. 93 is a side cross-sectional view of the glenoid implant 2200 at the cross-section 93-93 in FIG. 92.

The glenoid implant 2200 includes a baseplate 2210, a trunnion 2237 extending therefrom (e.g., for engaging a glenosphere), and a keel element 2230. The glenoid implant 2100 can include a central aperture 2240 therethrough configured to receive a central fastening element 2255 therethrough. The keel element 2230 is a single center wedge component. In such a configuration, more than one second fastening element 2215 does not pass through the keel element 2230 (see fig. 90), but goes directly into the scapula.

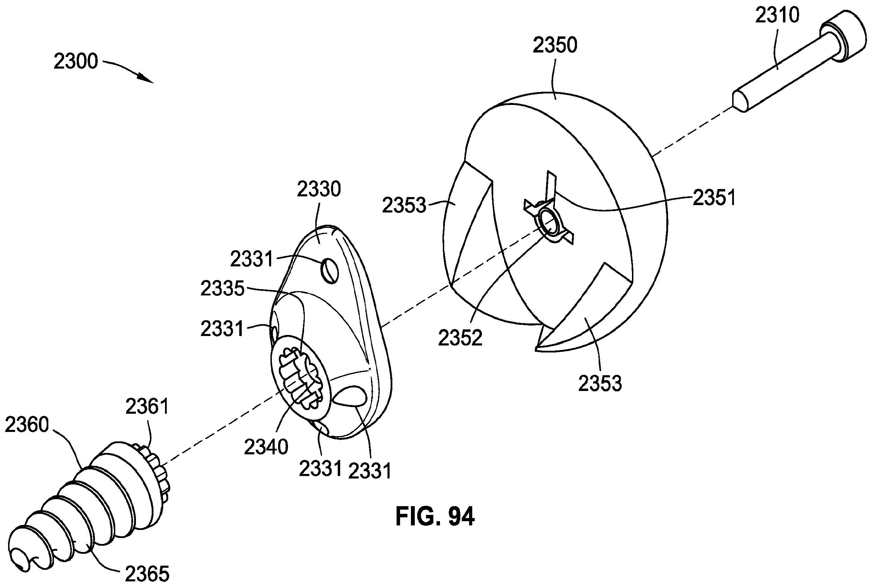

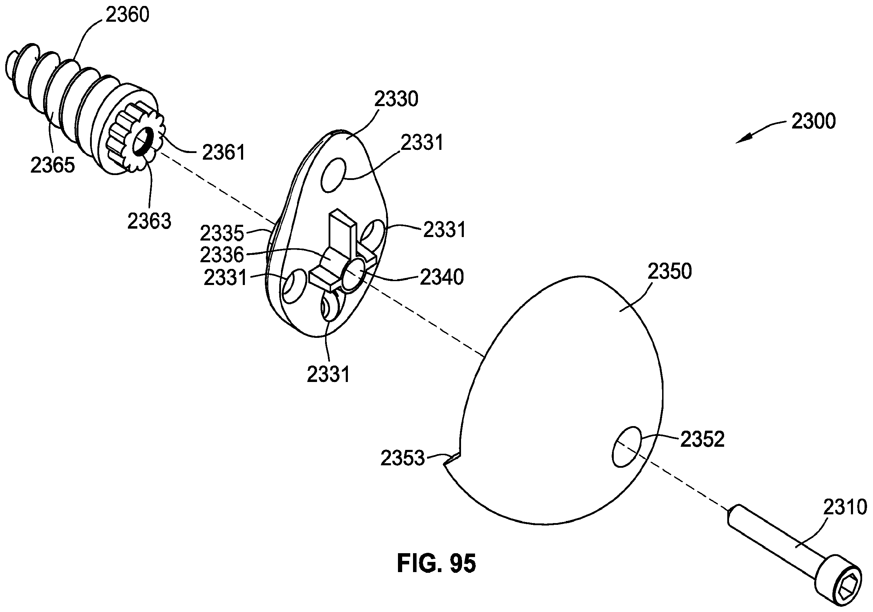

Referring generally to FIGS. 94-95, a glenoid implant 2300 is illustrated. FIGS. 94 and 95 are rear and front isometric exploded views of the glenoid implant 2300, respectively. The glenoid implant 2300 includes a reverse face plate 2330, a keel element 2360, a glenosphere 2350, and a central fastening element 2310. The reverse face plate 2330 includes a protrusion element 2336 (FIG. 95) with a first central aperture 2340 therethrough. The reverse face plate 2330 can also include a receiving element 2335 opposite the protrusion element 2336. The receiving element 2335 can have a raised profile. The reverse face plate 2330 can also include apertures 2331 therethrough. Each of the apertures 2331 are positioned around the central aperture 2340. The keel element 2360 can include a second central aperture 2363. The keel element 2360 can include a threaded element 2365 to aid in securing the keel element to a portion of a scapula. Furthermore, the keel element 2360 can include a locking element 2361 configured to couple with the receiving element 2335 of the reverse plate 2330. The glenosphere 2350 can include a third central aperture 2352. The glenosphere 2350 can also include a receiving space 2351 with a unique profile that corresponds with the protrusion element 2336 of the reverse face plate 2330.

The central fastening element 2310 can be configured to be at least partially positioned through the first central aperture 2340 of the reverse face plate 2330, the second central aperture 2363 of the keel element 2360, and the third central aperture 2352 of the glenosphere 2350. The central fastening element 2310 can aid in coupling the glenosphere 2350 to the reverse face plate 2330 and the keel element 2360 to the reverse face plate 2330.

In some implementations, the receiving element 2335 of the reverse face plate 2330 includes splines configured to non-rotationally couple with corresponding grooves in the connecting element 2361 of the keel element 2360. In alternative implementations, the receiving element 2335 of the reverse face plate 2330 includes grooves configured to non-rotationally couple with corresponding splines in the connecting element 2361 of the keel element 2360.

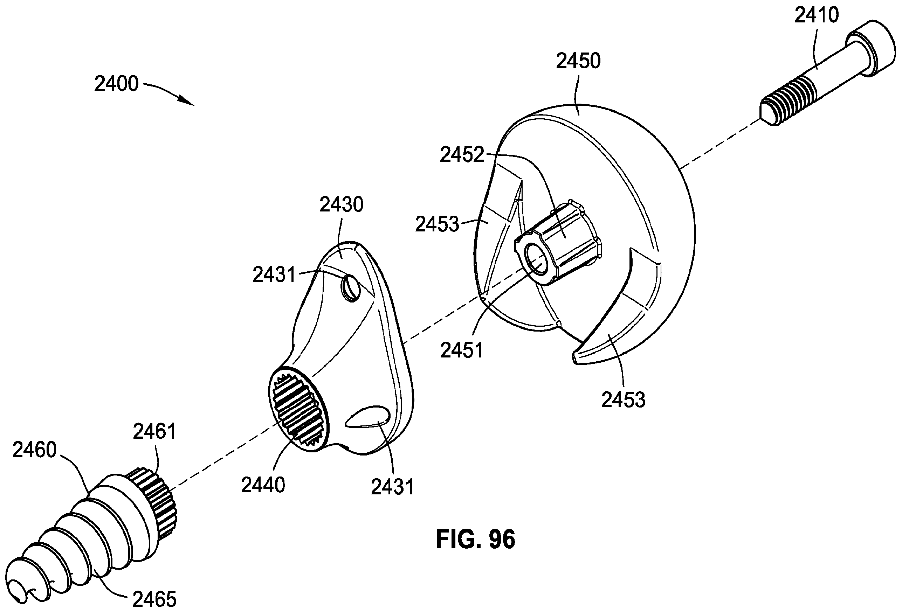

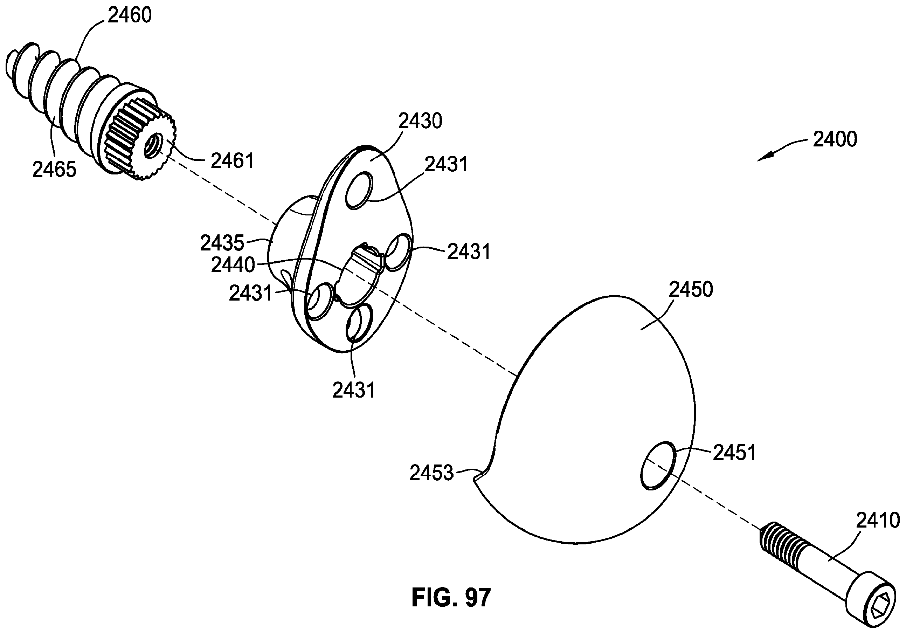

Referring generally to FIGS. 96-97, a glenoid implant 2400 is illustrated. FIGS. 96 and 97 are front and rear isometric exploded views of the glenoid implant 2400. The glenoid implant 2400 includes a reverse face plate 2430, a keel element 2460, a glenosphere 2450, and a central fastening element 2410, which are similar to the reverse face plate 2330, the keel element 2360, the glenosphere 2350, and the central fastening element 2310 of the glenoid implant 2300. The reverse face plate 2430 can include a receiving element 2440 in the place of the protrusion element 2336. The glenosphere 2450 can include a protrusion element 2452 configured to be received within the receiving element 2440 of the reverse face plate 2430. The glenosphere 2450 can also include wedge elements 2453 configured to abut the reverse face plate 2430 once coupled to the glenosphere 2450.

While the present disclosure has been described with reference to one or more particular embodiments or implementations, those skilled in the art will recognize that many changes may be made thereto without departing from the scope of the invention, which is defined by the claims.

## Claims

1. A glenoid implant (100), comprising:
a baseplate (140) having a central aperture (141) therethrough;
a first leg (110) extending from the baseplate (140) in a first direction, the first leg (110) having a first aperture (111) therein configured to receive a portion of a first fastening element (115) therethrough to aid in securing the first leg (110) to a first portion of a scapula of a patient,
the first leg (110) extending from the baseplate (140) generally along a first arc;
a second leg (120) extending from the baseplate (140) in a second direction, the second leg (120) having a second aperture (121) therein configured to receive a portion of a second fastening element (125) therethrough to aid in securing the second leg (120) to a second portion of the scapula of the patient,
the second leg (120) extending from the baseplate (140) generally along a second arc;
wherein a radius of curvature of the first arc is larger than a radius of curvature of the second arc; and
a third leg (130) extending from the baseplate (140) generally in a third direction, the third leg (130) having a third aperture (131) therein configured to receive a portion of a third fastening element (135) therethrough to aid in securing the third leg to a third portion of the scapula of the patient.

2. The glenoid implant of claim 1, wherein the baseplate, the first leg, the second leg, and the third leg form a single monolithic part.

3. The glenoid implant of claim 1, wherein the baseplate includes a first portion coupled to a second portion such that the central aperture is defined by both the first portion and the second portion.

4. The glenoid implant of claim 3, wherein the first portion of the baseplate, the first leg, and the second leg form a first monolithic part and wherein the second portion of the baseplate and the third leg form a second monolithic part that is separate and distinct from the first monolithic part.

5. The glenoid implant of any of claims 1-4, further comprising a central fastening element configured to be at least partially positioned through the central aperture of the baseplate to aid in securing the baseplate to a fourth portion of the scapula of the patient.

6. The glenoid implant of any of claims 1-5, wherein the third leg extends from the baseplate generally along a third arc.

7. The glenoid implant of claim 6, wherein a radius of curvature for the third arc is smaller than a radius of curvature for the second arc and a radius of curvature for the first arc, or wherein the radius of curvature for the third arc is larger than the radius of curvature for the second arc and the radius of curvature for the first arc.

8. The glenoid implant of any of claims 5-7, wherein a head (153) of the central fastening element is configured to be coupled with one or more additional components using a Morse taper press fit.

9. The glenoid implant of claim 8, wherein the head of the central fastening element is configured to be coupled with a glenosphere or a glenoid socket.

10. The glenoid implant of any of claims 1-5, wherein the first leg and the second leg each has a flat face surface and rounded rear surface configured to contact the scapula.

11. The glenoid implant of any of claims 1-5, wherein the first leg and the second leg each has a unique arc, and wherein each of the first leg and the second leg can be bent in place as desired depending on an anatomy of the scapula.

12. The glenoid implant of claim 10 or 11, wherein a first trunnion (336) extends from the first portion of the baseplate into the second portion of the baseplate.

13. The glenoid implant of claim 10 or 11, wherein a second trunnion (337) extending from the first portion of the baseplate is configured to be coupled with one or more additional components using a Morse taper press fit.

## Patentansprüche

1. Glenoidimplantat (100), umfassend:
eine Grundplatte (140) mit einer zentralen Öffnung (141);
einen ersten Schenkel (110), der sich von der Grundplatte (140) in einer ersten Richtung erstreckt, wobei der erste Schenkel (110) eine erste Öffnung (111) aufweist, die so ausgebildet ist, dass sie einen Abschnitt eines ersten Befestigungselements (115) durch sich hindurch aufnimmt, um die Befestigung des ersten Schenkels (110) an einem ersten Abschnitt eines Schulterblatts eines Patienten zu unterstützen,
wobei sich der erste Schenkel (110) von der Grundplatte (140) im Wesentlichen entlang eines ersten Bogens erstreckt;
einen zweiten Schenkel (120), der sich von der Grundplatte (140) in einer zweiten Richtung erstreckt, wobei der zweite Schenkel (120) eine zweite Öffnung (121) aufweist, die so ausgebildet ist, dass sie einen Abschnitt eines zweiten Befestigungselements (125) durch sich hindurch aufnimmt, um die Befestigung des zweiten Schenkels (120) an einem zweiten Abschnitt des Schulterblatts des Patienten zu unterstützen,
wobei sich der zweite Schenkel (120) von der Grundplatte (140) im Wesentlichen entlang eines zweiten Bogens erstreckt;
wobei der Krümmungsradius des ersten Bogens größer ist als der Krümmungsradius des zweiten Bogens; und
einen dritten Schenkel (130), der sich von der Grundplatte (140) im Wesentlichen in einer dritten Richtung erstreckt, wobei der dritte Schenkel (130) eine dritte Öffnung (131) aufweist, die so ausgebildet ist, dass sie einen Abschnitt eines dritten Befestigungselements (135) durch sich hindurch aufnimmt, um die Befestigung des dritten Schenkels an einem dritten Abschnitt des Schulterblatts des Patienten zu unterstützen.

2. Glenoidimplantat nach Anspruch 1, wobei die Grundplatte, der erste Schenkel, der zweite Schenkel und der dritte Schenkel ein einziges monolithisches Teil bilden.

3. Glenoidimplantat nach Anspruch 1, wobei die Grundplatte einen ersten Abschnitt aufweist, der mit einem zweiten Abschnitt so verbunden ist, dass die zentrale Öffnung sowohl durch den ersten Abschnitt als auch durch den zweiten Abschnitt definiert ist.

4. Glenoidimplantat nach Anspruch 3, wobei der erste Abschnitt der Grundplatte, der erste Schenkel und der zweite Schenkel ein erstes monolithisches Teil bilden und wobei der zweite Abschnitt der Grundplatte und der dritte Schenkel ein zweites monolithisches Teil bilden, das vom ersten monolithischen Teil getrennt und davon unterscheidbar ist.

5. Glenoidimplantat nach einem der Ansprüche 1 bis 4, ferner umfassend ein zentrales Befestigungselement, das so ausgebildet ist, dass es zumindest teilweise durch die zentrale Öffnung der Grundplatte geführt wird, um die Befestigung der Grundplatte an einem vierten Abschnitt des Schulterblatts des Patienten zu unterstützen.

6. Glenoidimplantat nach einem der Ansprüche 1 bis 5, wobei sich der dritte Schenkel von der Grundplatte im Wesentlichen entlang eines dritten Bogens erstreckt.

7. Glenoidimplantat nach Anspruch 6, wobei ein Krümmungsradius des dritten Bogens kleiner ist als ein Krümmungsradius des zweiten Bogens und ein Krümmungsradius des ersten Bogens oder wobei der Krümmungsradius des dritten Bogens größer ist als der Krümmungsradius des zweiten Bogens und der Krümmungsradius des ersten Bogens.

8. Glenoidimplantat nach einem der Ansprüche 5 bis 7, wobei ein Kopf (153) des zentralen Befestigungselements so ausgebildet ist, dass er mittels einer Morsekonus-Presspassung mit einer oder mehreren zusätzlichen Komponenten verbunden werden kann.

9. Glenoidimplantat nach Anspruch 8, wobei der Kopf des zentralen Befestigungselements so ausgebildet ist, dass er mit einer Glenosphäre oder einer Glenoidpfanne verbunden werden kann.

10. Glenoidimplantat nach einem der Ansprüche 1 bis 5, wobei der erste Schenkel und der zweite Schenkel jeweils eine flache Vorderseite und eine abgerundete Rückseite aufweisen, die so ausgebildet sind, dass sie mit dem Schulterblatt in Kontakt kommen.

11. Glenoidimplantat nach einem der Ansprüche 1 bis 5, wobei der erste Schenkel und der zweite Schenkel jeweils einen eigenen Bogen aufweisen und wobei sowohl der erste Schenkel als auch der zweite Schenkel je nach Anatomie des Schulterblatts nach Wunsch in situ gebogen werden können.

12. Glenoidimplantat nach Anspruch 10 oder 11, wobei sich ein erster Zapfen (336) vom ersten Abschnitt der Grundplatte in den zweiten Abschnitt der Grundplatte erstreckt.

13. Glenoidimplantat nach Anspruch 10 oder 11, wobei ein zweiter Zapfen (337), der sich vom ersten Abschnitt der Grundplatte erstreckt, so ausgebildet ist, dass er mittels einer Morsekonus-Presspassung mit einer oder mehreren zusätzlichen Komponenten verbunden werden kann.

## Revendications

1. Implant glénoïdien (100), comprenant:
une plaque de base (140) comportant une ouverture centrale (141) la traversant;
une première branche (110) s'étendant depuis la plaque de base (140) dans une première direction, la première branche (110) comportant une première ouverture (111) configurée pour recevoir une partie d'un premier élément de fixation (115) à travers celle-ci afin de faciliter la fixation de la première branche (110) à une première partie d'une omoplate d'un patient,
la première branche (110) s'étendant depuis la plaque de base (140) généralement le long d'un premier arc;
une deuxième branche (120) s'étendant depuis la plaque de base (140) dans une deuxième direction, la deuxième branche (120) comportant une deuxième ouverture (121) configurée pour recevoir une partie d'un deuxième élément de fixation (125) à travers celle-ci afin de faciliter la fixation de la deuxième branche (120) à une deuxième partie de l'omoplate du patient,
la deuxième branche (120) s'étendant depuis la plaque de base (140) généralement le long d'un deuxième arc; dans lequel le rayon de courbure du premier arc est supérieur au rayon de courbure du deuxième arc; et
une troisième branche (130) s'étendant depuis la plaque de base (140) généralement dans une troisième direction, la troisième branche (130) comportant une troisième ouverture (131) configurée pour recevoir une partie d'un troisième élément de fixation (135) à travers celle-ci afin de faciliter la fixation de la troisième branche à une troisième partie de l'omoplate du patient.

2. Implant glénoïdien selon la revendication 1, dans lequel la plaque de base, la première branche, la deuxième branche et la troisième branche forment une seule pièce monolithique.

3. Implant glénoïdien selon la revendication 1, dans lequel la plaque de base comprend une première partie couplée à une deuxième partie de telle sorte que l'ouverture centrale est définie à la fois par la première partie et la deuxième partie.

4. Implant glénoïdien selon la revendication 3, dans lequel la première partie de la plaque de base, la première branche et la deuxième branche forment une première pièce monolithique et dans lequel la deuxième partie de la plaque de base et la troisième branche forment une deuxième pièce monolithique qui est séparée et distincte de la première pièce monolithique.

5. Implant glénoïdien selon l'une quelconque des revendications 1 à 4, comprenant en outre un élément de fixation central configuré pour être au moins partiellement positionné à travers l'ouverture centrale de la plaque de base afin de faciliter la fixation de la plaque de base à une quatrième partie de l'omoplate du patient.

6. Implant glénoïdien selon l'une quelconque des revendications 1 à 5, dans lequel la troisième branche s'étend depuis la plaque de base généralement le long d'un troisième arc.

7. Implant glénoïdien selon la revendication 6, dans lequel le rayon de courbure du troisième arc est inférieur au rayon de courbure du deuxième arc et au rayon de courbure du premier arc, ou dans lequel le rayon de courbure du troisième arc est supérieur au rayon de courbure du deuxième arc et au rayon de courbure du premier arc.

8. Implant glénoïdien selon l'une quelconque des revendications 5 à 7, dans lequel une tête (153) de l'élément de fixation central est configurée pour être couplée à un ou plusieurs composants supplémentaires à l'aide d'un ajustement serré à cône de Morse.

9. Implant glénoïdien selon la revendication 8, dans lequel la tête de l'élément de fixation central est configurée pour être couplée à une glénosphère ou à une cavité glénoïdienne.

10. Implant glénoïdien selon l'une quelconque des revendications 1 à 5, dans lequel la première branche et la deuxième branche présentent chacune une surface frontale plane et une surface arrière arrondie configurées pour entrer en contact avec l'omoplate.

11. Implant glénoïdien selon l'une quelconque des revendications 1 à 5, dans lequel la première branche et la deuxième branche présentent chacune un arc unique, et dans lequel chacune de la première branche et de la deuxième branche peut être pliée en place comme souhaité en fonction de l'anatomie de l'omoplate.

12. Implant glénoïdien selon la revendication 10 ou 11, dans lequel un premier tourillon (336) s'étend depuis la première partie de la plaque de base vers la deuxième partie de la plaque de base.

13. Implant glénoïdien selon la revendication 10 ou 11, dans lequel un deuxième tourillon (337) s'étendant depuis la première partie de la plaque de base est configuré pour être couplé à un ou plusieurs composants supplémentaires à l'aide d'un ajustement serré à cône de Morse.
